# EUROPEAN PATENT SPECIFICATION

(11) **EP 2 177 907 B1**
(45) Date of publication and mention of the grant of the patent: **01.01.2014**
(21) Application number: 10000021.5
(22) Date of filing: 29.06.2005
(51) Int. Cl.: G01N 33/53, C12Q 1/37, C12Q 1/48, G01N 33/573

(54) **Indicator agent for noninflammatory stress response and use therefor**
Indikatormittel für nichtentzündliche Stressreaktion und Verwendung dafür
Agent indicateur d'une réponse au stress non inflammatoire et son utilisation

(30) Priority: 30.06.2004 JP 2004193712; 31.01.2005 JP 2005023978
(43) Date of publication of application: 21.04.2010
(62) Divisional of application: 05765312.3
(73) Proprietor: Sekiyama, Atsuo, Suita-shi, Osaka 5640063 (JP)
(72) Inventor: Sekiyama, Atsuo, Suita-shi, Osaka 5640063 (JP)
(74) Representative: Duckworth, Timothy John

(56) References cited:
- WO-A1-01/62285
- KALINA U ET AL: "IL-18 activates STAT3 in the natural killer cell line 92, augments cytotoxic activity, and mediates IFN-gamma production by the stress kinase p38 and by the extracellular regulated kinases p44erk-1 and p42erk-21." JOURNAL OF IMMUNOLOGY (BALTIMORE, MD. : 1950) 1 AUG 2000 LNKD- PUBMED:10903731, vol. 165, no. 3, 1 August 2000 (2000-08-01), pages 1307-1313, XP002583330 ISSN: 0022-1767
- WYMAN TRAVIS H ET AL: "Physiological levels of interleukin-18 stimulate multiple neutrophil functions through p38 MAP kinase activation" JOURNAL OF LEUKOCYTE BIOLOGY, vol. 72, no. 2, August 2002 (2002-08), pages 401-409, XP002583331 ISSN: 0741-5400
- KOKAI MASAHIRO ET AL: "The restraint stress-induced elevation in plasma interleukin-18 in mice. A special reference to an action of caspase-1" EUROPEAN CYTOKINE NETWORK, JOHN LIBBEY EUROTEXT LTD, FR, vol. 14, no. SUPPL.3, 1 September 2003 (2003-09-01), page 85, XP008086375 ISSN: 1148-5493
- KOKAI M ET AL: "Plasma interleukin-18 levels in patients with psychiatric disorders" JOURNAL OF IMMUNOTHERAPY, LIPPINCOTT WILLIAMS & WILKINS, HAGERSTOWN, MD, US LNKD- DOI:10.1097/00002371-200203001-00011, vol. 25, no. Supplement 1, 1 March 2002 (2002-03-01), page 6092, XP008086396 ISSN: 1524-9557
- SEKIYAMA ATSUO ET AL: "A stress-induced, superoxide-mediated caspase-1 activation pathway causes plasma IL-18 upregulation" IMMUNITY, CELL PRESS, US LNKD- DOI:10.1016/J.IMMUNI.2005.04.006, vol. 22, no. 6, 1 June 2005 (2005-06-01), pages 669-677, XP002474500 ISSN: 1074-7613
- HAN J ET AL: "A MAP kinase targeted by endotoxin and hyperosmolarity in mammalian cells." SCIENCE (NEW YORK, N.Y.) 5 AUG 1994 LNKD- PUBMED:7914033, vol. 265, no. 5173, 5 August 1994 (1994-08-05), pages 808-811, XP002583332 ISSN: 0036-8075
- ROBINSON M J ET AL: "Mitogen-activated protein kinase pathways", CURRENT OPINION IN CELL BIOLOGY, CURRENT SCIENCE, LONDON, GB, vol. 9, no. 2, 1 January 1997 (1997-01-01) , pages 180-186, XP002238980, ISSN: 0955-0674, DOI: 10.1016/S0955-0674(97)80061-0
- J RAINGEAUD ET AL: 'Pro-inflammatory cytokines and environmental stress cause p38 mitogen-activated protein kinase activation by dual phosphorylation on tyrosine and threonine.' THE JOURNAL OF BIOLOGICAL CHEMISTRY vol. 270, no. 13, 01 January 1995, pages 7420 - 7426, XP055038203
- WANG X S ET AL: "MOLECULAR CLONING AND CHARACTERIZATION OF A NOVEL P38 MITOGEN-ACTIVATED PROTEIN KINASE", JOURNAL OF BIOLOGICAL CHEMISTRY, AMERICAN SOCIETY FOR BIOCHEMISTRY AND MOLECULAR BIOLOGY, US, vol. 272, no. 38, 19 September 1997 (1997-09-19), pages 23668-23674, XP002053848, ISSN: 0021-9258, DOI: 10.1074/JBC.272.38.23668

## Description

### Technical Field

The present invention is directed to elucidating stress using molecular biology, thereby contributing to scientific development in the field of mental health.

### Background Art

Mental and/or physical stress influences host defense, including the nervous, endocrine and immune systems (see non-patent documents 1 and 2). Various cytokines (for example, IL-1β, IL-6, TNF-α and the like) undergo upregulation by stress (see non-patent documents 1 and 3). This suggests the possible involvement of cytokines in the interference of host defense (see non-patent document 4). However, the molecular mechanisms by which stress induces cytokines that damage host defense remain to be understood fully.

Interleukin-18 (IL-18) is a cytokine discovered as an interferon-γ (IFN-γ) inducing factor (see non-patent document 5 and patent document 1). IL-18 has a broad range of biological activities, including the induction of Fas ligands, the elevation of the cytolytic activity of T cells (see non-patent document 6) and the production of IL-4 and IL-13 (see non-patent document 7) (see non-patent document 8). IL-18 activates Toll-like receptor 2 (see non-patent document 9) and activates myelodifferentiation protein (Myd)-88 (see non-patent document 10). The activation thereof is essential to the induction of IL-6 (see non-patent document 11). Therefore, IL-18 is involved in the production of both the cytokines Th1 and Th2 (see non-patent document 12).

IL-18 is produced as a 24-kD precursor protein and processed into the 18-kD mature active type by IL-1β converting enzyme (ICE, or also referred to as caspase-1) (see non-patent document 13). Caspase-1 is induced as inactive type precursor protein procaspase-1 and activated by caspase-11 (see non-patent document 14). The expression of caspase-11 mRNA requires the trans-activation of NF-κB (see non-patent document 15), and this activation is reported to be mediated by P38MAP kinase (see non-patent document 16). The induction of caspase-11 mRNA by LPS (lipopolysaccharide) and the subsequent activation of caspase-1 are reported to be suppressed by SB203580, which is a P38MAP kinase inhibitor, in glyoma cell line C6 (see non-patent document 17).

Recent research has reported that IL-18 mRNA is expressed in the adrenal gland in response to adrenocorticotrophic hormone (ACTH) and cold stress (see non-patent document 18). Also, it has been reported that in the adrenal gland and immunocytes, IL-18 mRNA uses different promoters (see non-patent document 19). However, the induction of the mature type of IL-18 is not shown in either of the aforementioned two studies. On the other hand, in psychiatric patients, elevations of IL-18 in plasma have been reported (see non-patent document 20).

Han et al (1994) Science: 265; 808-811 discloses the cloning of a protein kinase, P38, that was tyrosine phosphorylated in response to LPS. Raingeaud et al (1995) the Journal of Biological Chemistry 270, 7420-7426 discloses that p38 is activated by treatment of cells with pro-inflammatory cytokines and environmental stress.

Robinson and Cobb (1997) Current Opinion in Cell Biology 9, 180-186 relates to mitogen-activated protein kinase pathways. This document discloses the identification of such pathways, the roles of these pathways in cells and cross-talk and signal integration among the pathways.

Kalina et al (2000) J. Immunol. 165, 1307-1313, investigates IL-18 mediated signal transduction using the human NK cell line NK92 as a model.

WO 01/62285 discloses the use of inhibitors of IL-18 in the preparation of a medicament for treatment and/or prevention of liver injury. This document also discloses the use of IL-18 inhibitors in the preparation of a medicament for treatment and/or prevention of arthritis. Furthermore, WO 01/62285 discloses these inhibitors for the preparation of a medicament for treatment and/or prevention of inflammatory bowel diseases.

Wyman et al (2002) Journal of Leukocyte Biology; 72: 401-409 investigates IL-18 signalling in neutrophils. At physiological concentrations, IL-18 was shown to be an effective neutrophil priming agent.

Kokai *et al* (2003) European Cytokine Network 14 (Suppl.), 85, discloses that elevated plasma IL-18 levels were observed in drug-naive patients with depression and anxiety disorder. This document also discloses that plasma IL-18 was elevated when mice were subjected to 6-hours restraint stress.

In modem society, humans are working and living while being exposed to various forms of stress. Generally, there are individual differences in how stress is felt, with no definite index available for the presence or absence and intensity of stress. Research to date has not shown whether or not stress results in increases in cytokines such as IL-18, and whether or not IL-18 plays a role in the disorder of host defense induced by stress.
Patent document 1: Japanese Patent Unexamined Publication No. HEI-8-193098
Non-patent document 1: Dugue, B. et al. Scand. J. Clin. Invest. 53, 555-561 (1993)
Non-patent document 2: Kiecolt-Glaser, J.K. et al. Proc. Natl. Acad. Sci. USA 93, 3043-3047(1996)
Non-patent document 3: Endocrinology 133, 2523-2530 (1993)
Non-patent document 4: Schubert, C. et al. Psychosom. Med. 61, 876-882 (1999)
Non-patent document 5: Zhou, D. et al. Nature 378, 88-91 (1995)
Non-patent document 6: Nakanishi, K. et al. Annu. Rev. Immunol. 19 423-474 (2001)
Non-patent document 7: Hoshino, T. et al. J. Immunol. 162, 5070-5077 (1999)
Non-patent document 8: Dinarello, C.A. et al. J. Leukoc. Biol. 63, 658-664 (1998)
Non-patent document 9: Blease, K. et al. Inflamm. Res. 50, 552-560 (2001)
Non-patent document 10: Adachi, O. et al. Immunity 9, 143-150 (1998)
Non-patent document 11: Takeuchi, O. et al. J. Immunol. 165, 5392-5396 (2000)
Non-patent document 12: Hoshino, T. et al. J. Immunol. 166, 7014-7018 (2001)
Non-patent document 13: Gu, Y. et al. Science 275, 206-209 (1997)
Non-patent document 14: Wang, S. et al. Cell 92, 501-509 (1998)
Non-patent document 15: Schauvliege, R. et al. J. Biol. Chem. 277, 41624-41630 (2002)
Non-patent document 16: Vanden Berghe, W. et al. J. Biol. Chem. 273, 3285-3290 (1998)
Non-patent document 17: Hur, J. et al. FEBS Lett. 507, 157-162 (2001)
Non-patent document 18: Conti, B. et al. J. Biol. Chem. 272, 2035-2037 (1997)
Non-patent document 19: Suganuma, S. et al. J. Immunol. 165, 6287-6292 (2000)
Non-patent document 20: Kokai, M. et al. J. Immunother. 25, 68-71 (2002)

### Disclosure of the Invention

### Problems to Be Solved by the Invention

It is an object of the present invention to provide a system capable of visualizing and quantitatively detecting events in a living organism exposed to stress using molecular biology. It is another object of the present invention to provide a means that enables the management and avoidance of physical disorders due to stress.

### Means of Solving the Problems

The present inventor conducted research with a focus on the processing of IL-18 precursor protein and the release of mature type IL-18 into plasma in examining the expression of cytokines in mice undergoing restraint stress, found that an cascade in a living organism to activate IL-18 is induced by stress, and accomplished the present invention.

Accordingly, the present invention relates to:
[1] a method of measuring the degree of stress excluding inflammatory stress, which comprises measuring an increase in the amount or activity of IL-18 and an increase in the activity of P3 8MAP kinase using an IL-18 antibody and a P38MAP kinase antibody as a visualizing agent, wherein the amount or activity of IL-18 is measured in adrenal tissue or blood collected from an animal and the activity of P38MAP kinase is measured in adrenal tissue collected from an animal;
[2] the method described in [1], wherein the IL-18 is active type IL-18 present in blood, the amount or activity of IL-18 in blood is measured and/or the P38MAP kinase is phosphorylated kinase;
[3] the method described in [2], wherein the P38MAP kinase is derived from a mouse, and the phosphorylation site of the kinase is Thr180 or Tyr182, or the P38MAP kinase is derived from a human, and the phosphorylation site of the kinase is Thr185 or Tyr187;
[4] the method described in any one of [1] to [3], wherein the stress is mental stress, optionally restraint stress;
[5] the method of described in any one of [1] to [4], wherein the animal is a human;
[6] use of a visualizing agent which comprises an IL-18 antibody and a visualizing agent which comprises a P38MAP kinase antibody in detecting a response to stress excluding inflammatory stress by detecting an increase in the amount or activity of IL-18 and an increase in the activity of P38MAP kinase, wherein the amount or activity of IL-18 is detected in blood or adrenal tissue collected from an animal and the activity of P38MAP kinase is detected in adrenal tissue collected from an animal;
[7] the use of [6], wherein the IL-18 antibody specifically recognizes active type IL-18 and/or the P38MAP kinase antibody is an anti-phosphorylated P38MAP kinase antibody;
[8] the use of [7], wherein the anti-phosphorylated P38MAP kinase antibody recognizes a P38MAP kinase having phosphorylated Thr180 or Tyr182, or a P38MAP kinase having phosphorylated Thr185 or Tyr187;
[9] use of a kit in a method of measuring stress excluding inflammatory stress described in any one of [1] to [5], which kit comprises the visualizing agents described in any one of [6] to [8];
[10] a method of screening for a substance having a therapeutic effect, ameliorating effect or prophylactic effect on a disease that occurs, exacerbates or recurs due to mental stress, which comprises:
   1) quantifying the amount or activity of IL-18 in adrenal tissue or blood collected from an animal and the activity of P38MAP kinase in adrenal tissue collected from the animal;
   2) comparing the amount or activity of said IL-18 and the activity of said P38MAP kinase in the presence and absence of the test substance; and
   3) judging that a test substance that reduces or increases the amount or activity of IL-18 and the activity of P38MAP kinase is a substance having a therapeutic effect, ameliorating effect or prophylactic effect on a disease that occurs, exacerbates or recurs due to mental stress;
   optionally wherein the disease is systemic lupus erythematosus, lupus nephritis, atopic dermatitis, multiple sclerosis, bronchial asthma, psoriasis, cerebral ischemia, cerebral degeneration, encephalitis, articular rheumatism, emmeniopathy, endometriosis or sexual desire reduction;
[11] a method of screening for a substance having a suppressive effect or preventive effect on oxidative stress, which comprises:
   1) quantifying the amount or activity of IL-18 in adrenal tissue or blood collected from an animal and the activity of P38MAP kinase in adrenal tissue collected from the animal;
   2) comparing the amount or activity of said IL-18 and the activity of said P38MAP kinase in the presence and absence of the test substance; and
   3) judging that a test substance that reduces or increases the amount or activity of IL-18 and the activity of P38MAP kinase is a substance having a suppressive effect or preventative effect on oxidative stress; and
[12] the method described in [10] or [11], wherein the IL-18 is active-type IL-18 and/or the P38MAP kinase is phosphorylated kinase.

### EFFECT OF THE INVENTION

The levels of IL-18 components and a cascade that has P38MAP kinase as the starting point and that is mediated by superoxide produced due to non-inflammatory stress (hereinafter referred to as the stress cascade) have been clarified. This is helpful as an index not only for the degree of non-inflammatory stress to which each living organism undergoes, but also for the evaluation or prediction of the risk of collapse of host defense.

In the method of the present invention, IL-18 and P38MAP kinase are acting as an indicator agent. Because it contains ingredients involved in the stress cascade, the indicator agent can be utilized for screening of a substance that reduces these ingredients and activities, and is helpful in developing pharmaceuticals and foods with health claims (foods for specified health uses and foods with nutrient function claims) comprising such a substance as an active ingredient.

An IL-18 antibody and a P38MAP kinase antibody constitute visualizing agents in the present invention. By means of these visualising agents, the levels of IL-18 and the stress cascade can be quantified, and it is possible to open a new way to controlling non-inflammatory stress, which has been difficult to control. Furthermore, use of the visualizing agent in the present invention is helpful in the elucidation of pathology, drug innovation development, and therapeutic model development.

According to the method of the present invention of measuring the degree of non-inflammatory stress, the degree of stress to which each living organism undergoes can easily be quantified, and it is particularly useful for taking measures considering laborers' mental health.

### Brief Description of the Drawings

[Fig. 1A] A drawing showing plasma IL-18 and ACTH levels undergoing upregulation by restraint stress.
[Fig. 1B] A drawing showing that plasma IL-18 levels undergoing upregulation by restraint stress are suppressed by anti-ACTH antibody or dexamethasone.
[Fig. 1C] A drawing showing that IL-18 in the adrenal undergoing upregulation by restraint stress is suppressed by anti-ACTH antibody or dexamethasone.
[Fig. 1D] A drawing comparing IL-18 in plasma after administration of ACTH and IL-18 in plasma after restraint stress loading.
[Fig. 1E] A drawing comparing IL-18 in the adrenal after administration of ACTH and IL-18 in the adrenal after restraint stress loading.
[Fig. 1F] A drawing showing the results of an examination of IL-18 protein in the adrenal (lanes 1 to 3) and plasma (lanes 4 to 6) by Western blotting. Lanes 1 and 4 show the results obtained with control, lanes 2 and 5 show the results obtained after ACTH was administered, and lanes 3 and 6 show the results obtained after restraint stress was loaded.
[Fig. 2A] A drawing showing the results of an examination of procaspase-1 in the adrenal by Western blotting. Lane 1 shows the results obtained with control, lane 2 shows the results obtained after ACTH administration, and lane 3 shows the results obtained after restraint stress loading.
[Fig. 2B] A drawing showing that caspase-1 activity in the adrenal undergoing upregulation by restraint stress is suppressed by the caspase-1 inhibitor YVAD-CHO.
[Fig. 2C] A drawing showing that IL-18 in plasma undergoing upregulation by restraint stress is suppressed by YVAD-CHO.
[Fig. 2D] A drawing showing that IL-18 in the adrenal undergoing upregulation by restraint stress is not suppressed by YVAD-CHO.
[Fig. 2E] A drawing showing that caspase-1 activity in the adrenal undergoing upregulation by restraint stress is suppressed by the caspase-11 inhibitor Z-FA-fmk.
[Fig. 2F] A drawing showing that IL-18 activity in plasma undergoing upregulation by restraint stress is suppressed by the caspase-11 inhibitor Z-FA-fmk.
[Fig. 3A] A drawing showing that the caspase-11 precursor undergoing upregulation by restraint stress is suppressed by SB203580, which is a P38MAP kinase inhibitor.
[Fig. 3B] A drawing showing that caspase-1 activity induced by restraint stress is suppressed by SB203580.
[Fig. 3C] A drawing showing that IL-18 in plasma induced by restraint stress is suppressed by SB203580.
[Fig. 3D] A drawing showing the results of an examination of phosphorylated P38MAP kinase in the adrenal by Western blotting. Lane 1 shows the results obtained without stress, lane 2 shows the results obtained with stress loading, lane 3 shows the results obtained with the addition of YVAD-CHO, lane 4 shows the results obtained with the addition of Z-FA-fmk, and lane 5 shows the results obtained in the case of stress-loaded caspase-1 KO mice.
[Fig. 3E] A photograph showing the results of staining of the adrenal of a stress-loaded mouse using immunohistochemistry. In the overlapping region in the zona reticularis of adrenal cortex, the induction of IL-18 (panel 1), pro/caspase-1 (panel 2), pro/caspase-11 (panel 3), and activated (phosphorylated) P38MAP kinase (panel 4) is shown.
[Fig. 3F] A photograph showing the expression of activated (phosphorylated) P38MAP kinase in the presence of caspase inhibitor. Panel 1 shows the results obtained with the addition of Z-FA-fmk, and panel 2 shows the results obtained with the addition of YVAD-CHO.
[Fig. 4A] A drawing showing that activated (phosphorylated) P38MAP kinase undergoing upregulation by restraint stress is suppressed by SOD.
[Fig. 4B] A drawing showing that procaspase-11 undergoing upregulation by restraint stress is suppressed by SOD.
[Fig. 4C] A drawing showing that caspase-1 activity induced by restraint stress is suppressed by SOD.
[Fig. 4D] A drawing showing that IL-18 in plasma undergoing upregulation by restraint stress is suppressed by SOD.
[Fig. 4E] A drawing showing that IL-18 in the adrenal undergoing upregulation by restraint stress is not suppressed by SOD.
[Fig. 4F] A drawing showing that IL-18 in plasma undergoing upregulation by restraint stress is suppressed by DPI.
[Fig. 5A] A drawing showing plasma IL-6 levels in stress-loaded mice.
[Fig. 5B] A drawing showing that in stress-loaded wild type mice, plasma IL-6 levels undergoing upregulation are suppressed by caspase-1 inhibitor, SOD and anti-IL-18 antibody.
[Fig. 6] A drawing showing that in MRL/lpr mice, the exacerbation of lupus nephritis due to stress loading is suppressed by administration of anti-IL-6 antibody or anti-IL-18 receptor antibody.
[Fig. 7A] A drawing showing the effect of a suppressant of superoxide (SOD) on IL-18 release from keratinocytes with paraquat stimulation.
[Fig. 7B] A drawing showing the effect of a suppressant of superoxide (vitamin E) on IL-18 release from keratinocytes with paraquat stimulation.
[Fig. 7C] A drawing showing the effect of a suppressant of superoxide (SOD) on IL-18 release from keratinocytes with UV-B ray irradiation.
[Fig. 7D] A drawing showing the effect of a suppressant of superoxide (vitamin E) on I-18 release from keratinocytes with UV-B ray irradiation.
[Fig. 8A] A drawing showing the effect of a suppressant of superoxide (SOD) on IL-18 release from microglia cells with paraquat stimulation.
[Fig. 8B] A drawing showing the effect of a suppressant of superoxide (vitamin E) on IL-18 release from microglia with paraquat stimulation.
[Fig. 9A] A drawing showing the effect of a suppressant of superoxide (SOD) on IL-18 release from astrocytes with paraquat stimulation.
[Fig. 9B] A drawing showing the effect of a suppressant of superoxide (vitamin E) on IL-18 release from astrocytes with paraquat stimulation.
[Fig. 10A] A drawing showing the effect of a suppressant of superoxide (SOD) on IL-18 release from macrophages with paraquat stimulation.
[Fig. 10B] A drawing showing the effect of a suppressant of superoxide (vitamin E) on IL-18 release from macrophages with paraquat stimulation.
[Fig. 11A] A drawing showing the effect of a suppressant of superoxide (SOD) on IL-18 release from synovial membrane tissue with paraquat stimulation.
[Fig. 11B] A drawing showing the effect of a suppressant of superoxide (vitamin E) on IL-18 release from synovial membrane tissue with paraquat stimulation.
[Fig. 12A] A drawing showing the effect of a suppressant of superoxide (SOD) on IL-18 release from renal tubular epithelial cells with paraquat stimulation.
[Fig. 12B] A drawing showing the effect of a suppressant of superoxide (vitamin E) on IL-18 release from renal tubular epithelial cells with paraquat stimulation.
[Fig. 13A] A drawing showing changes in plasma ACTH concentration caused by IL-18 administration.
[Fig. 13B] A drawing showing changes in plasma FSH concentration caused by IL-18 administration.
[Fig. 13C] A drawing showing changes in plasma LH concentration caused by IL-18 administration.

### Best Mode for Embodying the Invention

The present invention is based on the finding of a novel cascade mediated by superoxide induced by non-inflammatory stress (stress cascade), making it possible to visualize and manage non-inflammatory stress.

Abbreviations for amino acids, (poly)peptides, (poly)nucleotides and the like hereinafter used in the present description are based on the IUPAC-IUB rules [IUPAC-IUB Communication on Biological Nomenclature, Eur. J. Biochem., 138:9 (1984)], "Guideline for the Preparation of Descriptions etc. Including Base Sequences or Amino Acid Sequences" (edited by the Japan Patent Office), and abbreviations in common use in relevant fields.

"Proteins" or "(poly)peptides" as used herein include not only "proteins" or "(poly)peptides" shown by particular amino acid sequences (SEQ ID NO:2, 4, 6, 8 or 10), but also homologous forms (homologues and splice variants), mutants, derivatives, mature forms and amino acid modifications thereof and the like, as long as they are equivalent thereto in terms of biological function. Here, as examples of the homologues, proteins of other biological species such as mice and rats, corresponding to human proteins, can be mentioned, and these can be deductively identified from the base sequences of genes identified by HomoloGene (http://www.ncbi.nlm.nih.gov/HomoloGene/). Also, the mutants include naturally occurring allele mutants, non-naturally-occurring mutants, and mutants having an amino acid sequence altered by artificially deleting, substituting, adding, or inserting an amino acid. Note that as the above-described mutants, those having a homology of at least 70%, preferably 80%, more preferably 95%, still more preferably 97%, to mutation-free proteins or (poly)peptides, can be mentioned. Also, the amino acid modifications include naturally occurring amino acid modifications and non-naturally-occurring amino acid modifications, and specifically, phosphorylated forms of amino acids can be mentioned.

Accordingly, the term "IL-18 protein" or simply "IL-18" as used herein, unless otherwise specified by a sequence identification number, is intended to include human IL-18, which is shown by a particular amino acid sequence (SEQ ID NO:2), homologous forms, mutants, derivatives, mature forms and amino acid modifications thereof and the like. Specifically, human IL-18, which has the amino acid sequence shown by SEQ ID NO:2 (GenBank Accession No. NM_001562), mouse homologues and rat homologues thereof and the like are included.

Also, the term "P38MAP kinase protein" or simply "P38MAP kinase" as used herein, unless otherwise specified by a sequence identification number, is intended to include human P38MAP kinase, which is shown by a particular amino acid sequence (SEQ ID NO:4), or homologous forms, mutants, derivatives, mature forms and amino acid modifications thereof and the like. Specifically, human P38MAP kinase, which has the amino acid sequence shown by SEQ ID NO:4 (GenBank Accession No. NM_138957), mouse homologues and rat homologues thereof and the like are included.

Also, the term "caspase-11 protein" or simply "caspase-11" as used herein, unless otherwise specified by a sequence identification number, is intended to include mouse caspase-11, which is shown by a particular amino acid sequence (SEQ ID NO:6), and homologous forms, mutants, derivatives, mature forms and amino acid modifications thereof and the like. Specifically, mouse caspase-11, which has the amino acid sequence shown by SEQ ID NO:6 (GenBank Accession No. MMCASP11), human homologues and rat homologues thereof and the like are included.

Also, the term "caspase-1 protein" or simply "caspase-1" as used herein, unless otherwise specified by a sequence identification number, is intended to include human caspase-1, which is shown by a particular amino acid sequence (SEQ ID NO:8), homologous forms, mutants, derivatives, mature forms and amino acid modifications thereof and the like. Specifically, human caspase-1, which has the amino acid sequence shown by SEQ ID NO:8 (GenBank Accession No. NM_033292), mouse homologues and rat homologues thereof and the like are included.

Also, the term "IL-6 protein" or simply "IL-6" as used herein, unless otherwise specified by a sequence identification number, is intended to include human IL-6, which is shown by a particular amino acid sequence (SEQ ID NO:10), homologous forms, mutants, derivatives, mature forms and amino acid modifications thereof and the like. Specifically, human IL-6, which has the amino acid sequence shown by SEQ ID NO:10 (GenBank Accession No. NM_000600), mouse homologues and rat homologues thereof and the like are included.

In the present invention, "antibodies" include polyclonal antibodies, monoclonal antibodies, chimeric antibodies, single-stranded antibodies, or a part of the above-described antibodies having antigen bindability, such as Fab fragments and fragments produced with an Fab expression library.

Also, in the present invention, "a body defense system" denotes a defense system by immune system function, a defense system by nervous/endocrine system function, particularly a defense system by the cerebral cortex-hypothalamus-pituitary-adrenocortical system and the autonomic nervous system, and a host defense system by the nervous, endocrine and immune systems mediated by cytokines.

"Non-inflammatory stress" as used herein refers to stress excluding inflammatory stress due to infection, tumors and the like, includes what is called oxidative stress, which is mediated by superoxide, and is hereinafter sometimes abbreviated as "stress". As the non-inflammatory stress, specifically, physical stress due to high temperature, low temperature, high pressure, low pressure, noise, radiation, ultraviolet and the like, hypoxia, chemical stress due to harmful chemical substances such as heavy metals and arsenic, and mental stress due to anger, anxiety, fear, tension, restraint and the like, can be mentioned as examples, and in the present invention, physical stress, chemical stress, or mental stress is preferable; as the mental stress, restraint stress is more preferable, and restraint stress for a long time is particularly preferable. Here, a long time, not generically defined because of organism and individual differences, can be exemplified by 6 hours or more in the case of an animal. As the physical stress, stress due to ultraviolet irradiation is preferable; as the chemical stress, stress due to harmful chemical substance exposure, particularly stress due to paraquat exposure, is preferable.

"Non-inflammatory stress response" as used herein refers to an in vivo reaction of a living organism exposed to non-inflammatory stress to the stress.

"Superoxide" as used herein refers to a kind of active oxygen produced in a living organism, represented by superoxide anions or radicals, and is known to be eliminated by superoxide dismutase (SOD), or to react with other substances, in the body, and is used in the present invention with a meaning including the metabolites of superoxide.

The "indicator agent" serves as an index for non-inflammatory stress responses mediated by superoxide, and has been developed by the discovery of "the cytokine cascade in non-inflammatory stress (referred to as the stress cascade)", which was for the first time demonstrated by the present inventor on the basis of the findings described below.

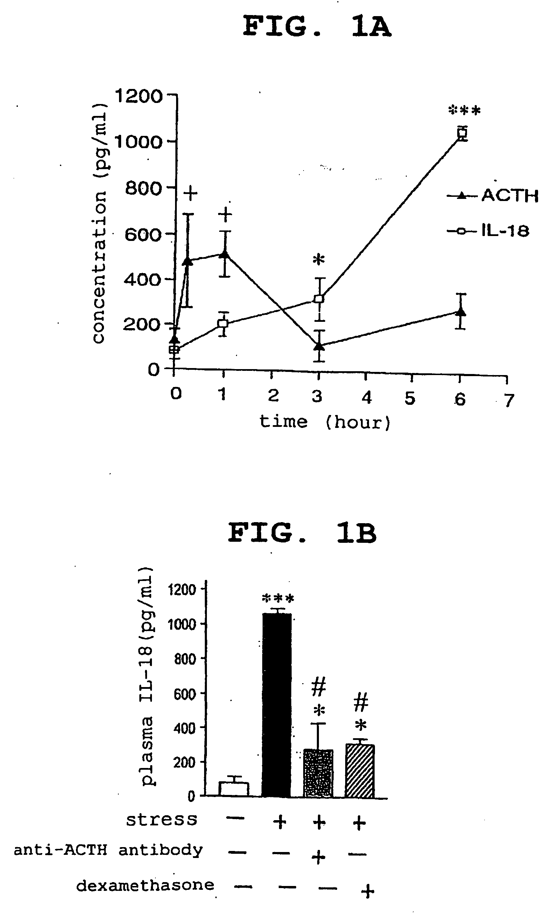

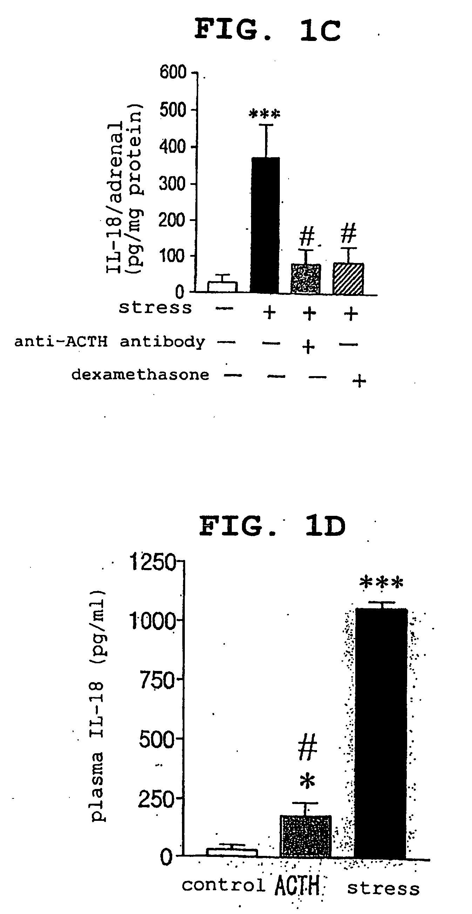

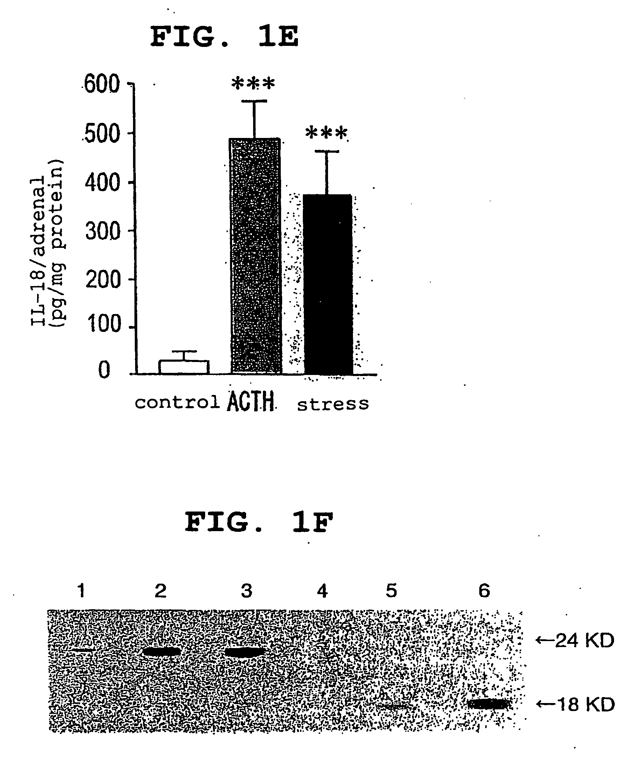

Giving restraint stress to an animal increases the level of the 24-kD IL-18 precursor protein in the adrenal, and the level of the 18-kD mature type in plasma (Fig. 1). These increases occur after adrenocorticotrophic hormone (ACTH) in plasma is elevated by the stress. This shows that in the adrenal, ACTH is involved in the induction of IL-18 precursor. This is supported by the discoveries that administration of anti-ACTH antiserum inhibits the increase in the levels of both mature type and precursor type IL-18, and that ACTH release induced by stress is inhibited by dexamethasone, whereby mature type and precursor type IL-18 are also inhibited. These results show that restraint stress induces ACTH, and then induces IL-18 precursor in the adrenal.

In the adrenal of a stress-loaded animal, not only IL-18 precursor protein but also mature type IL-18 is induced (Fig. 1F), suggesting that conversion from the precursor type to the mature type might occur due to the stress. Inhibiting the induction by ACTH of IL-18 precursor in the adrenal leads to the suppression of plasma IL-18 level. In summary, it is suggested that the IL-18 precursor in the adrenal induced by ACTH may be converted to the mature type and secreted in plasma. The conversion of the IL-18 precursor to the mature type is an important regulatory process in the accumulation of mature IL-18 in plasma in stress-loaded animals.

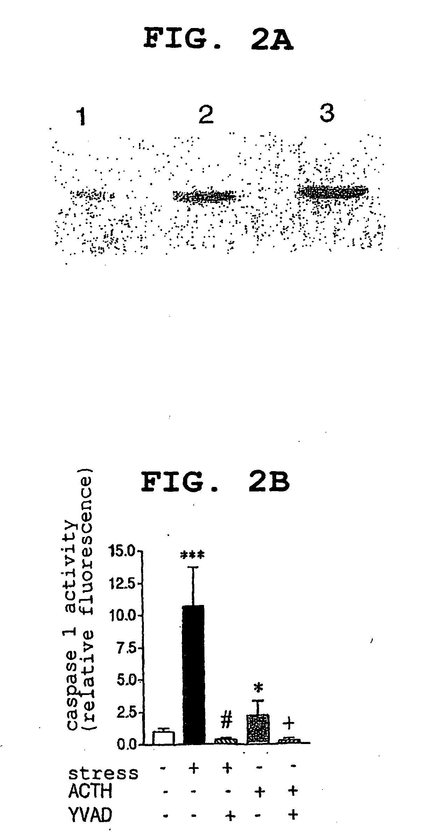

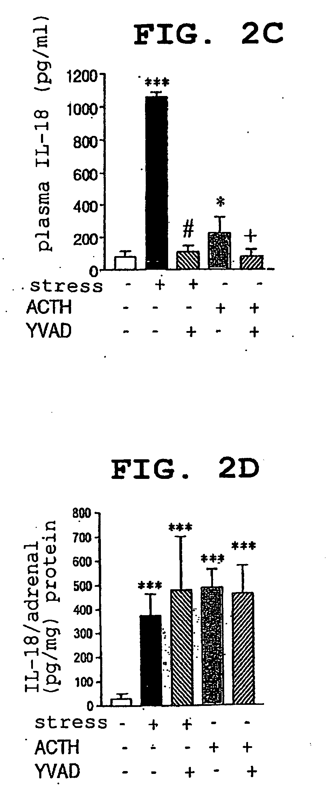

Caspase-1 precursor and the activity thereof are reported to be induced by LPS in the adrenal. In the present invention, it was shown that caspase-1 precursor was also induced by stress in the adrenal (Fig. 2A). YVAD-CHO, which is a caspase-1 inhibitor, inhibits the elevation of plasma IL-18 (Fig. 2B) but does not inhibit the induction of IL-18 precursor in the adrenal (Fig. 2D). In stress-loaded caspase-1 knockout (KO) animals, IL-18 precursor undergoes upregulation in the adrenal, but plasma IL-18 was undetectable. From these results, it is suggested that the caspase-1 induced by stress may have resulted in the processing of IL-18 precursor in the adrenal with respect to the accumulation of mature IL-18 in plasma.

Caspase-11 is reported to activate caspase-1. In the present invention, because Z-FA-fmk, which is an inhibitor of caspase-11, inhibited the activation of caspase-1 undergoing activation by stress, the involvement of caspase-11 in caspase-1 activation in stress-loaded animals is suggested. It has been reported that the induction of caspase-11 precursor requires the trans-activation of NF-κB, and that P38MAP kinase mediates the trans-activation of NF-κB. Furthermore, it has been reported that SB203580, which is a P38MAP kinase inhibitor, downregulates the induction of caspase-11.

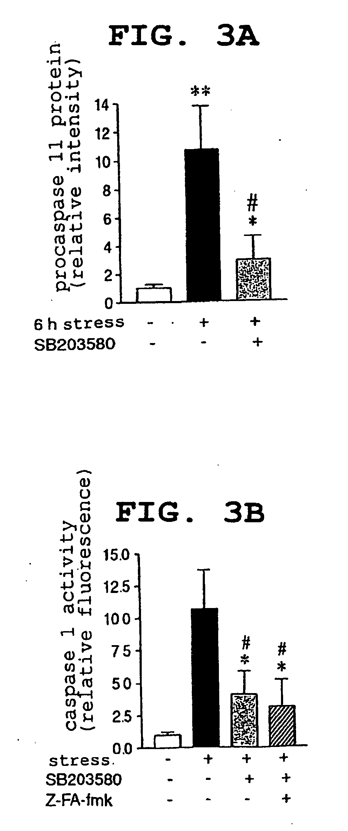

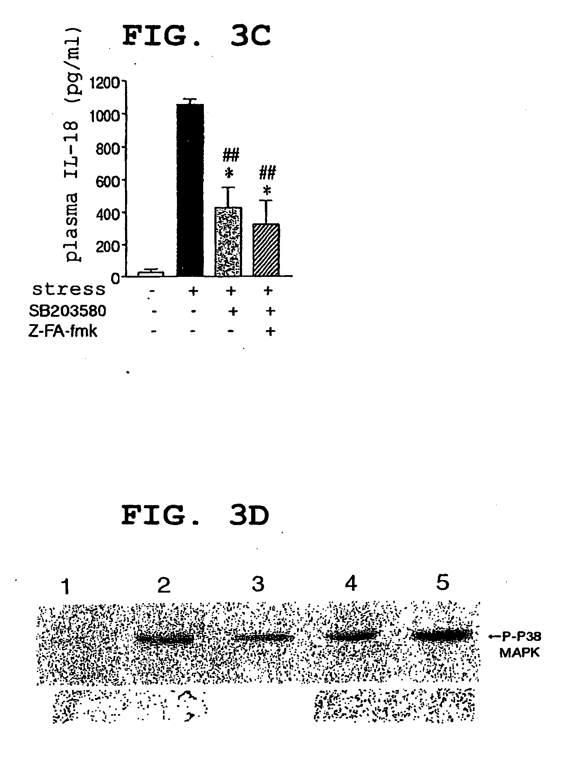

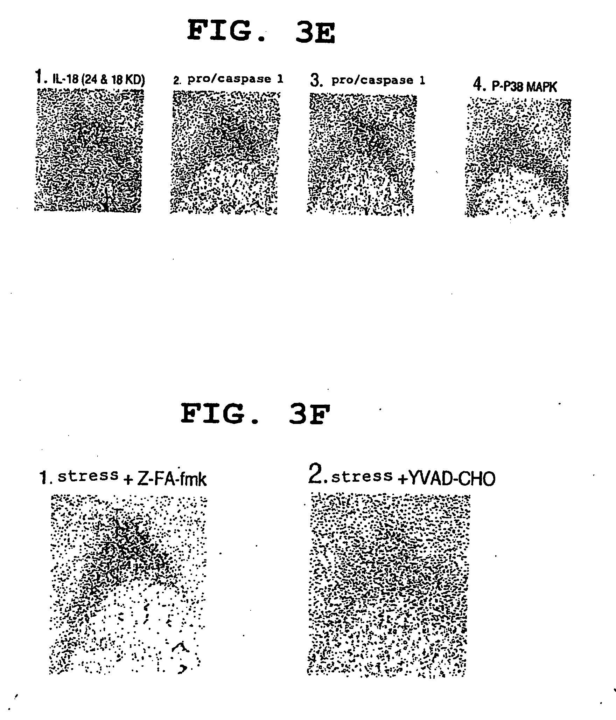

The present inventor discovered that the aforementioned SB203580 inhibited the stress-induced increases in the protein level of caspase-11 (Fig. 3A), caspase-1 activity (Fig. 3B) and plasma IL-18 level (Fig. 3C), respectively. The caspase-1 inhibitor YVAD-CHO and the caspase-11 inhibitor Z-FA-fmk suppress the stress-induced elevation of plasma IL-18 level without influencing the phosphorylation of P38MAP kinase (Figs. 3D and 3F). The stress-induced phosphorylation of P38MAP kinase protein occurred both in caspase-1 KO animals and in wild type animals (Fig. 3C). From these results, it is suggested that P38MAP kinase may be present upstream of caspase-11, form the P38MAP kinase → caspase-11 → caspase-1 cascade, and result in conversion from IL-18 precursor to the mature type in the adrenal, and release active type (mature type) IL-18 into plasma in stress-loaded animals.

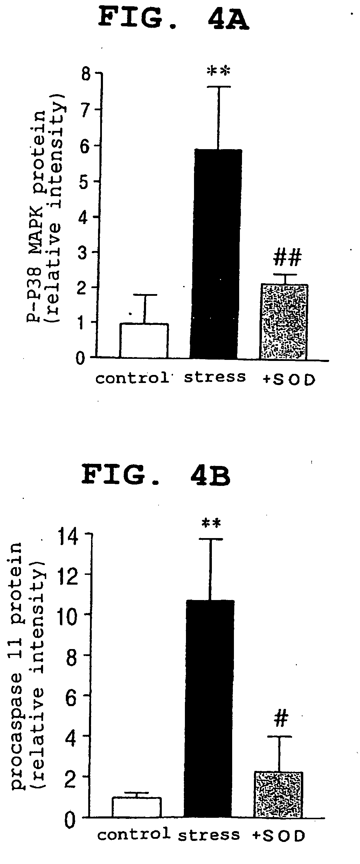

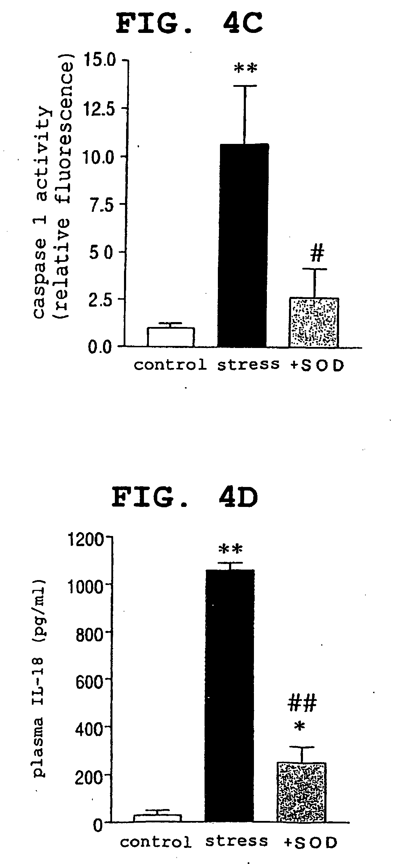

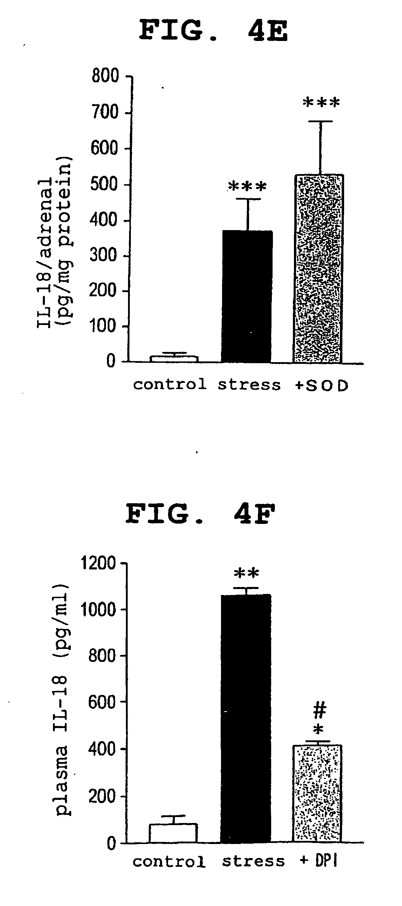

Active oxygen species are reported to mediate the phosphorylation of P38MAP kinase in pulmonary fibroblasts, neutrophils and monocytes. In the present invention, superoxide dismutase (SOD) inhibited the activation of P38MAP kinase (Fig. 4A), the induction of caspase-11, the activation of caspase-1 in the adrenal, and the elevation of plasma IL-18 level (Figs. 4B, C, D). However, the induction of IL-18 precursor in the adrenal was not influenced by SOD (Fig. 4E). From these results, it is suggested that stress may induce superoxide, which in turn may activate the P38MAP kinase cascade. Therefore, the process of generation of IL-18 in plasma in stress-loaded animals can be summarized as described below. Stress activates the hypothalamus-pituitary-adrenal (HPA) axis to induce ACTH. Also, stress generates superoxide, which in turn induces the phosphorylation of P38MAP kinase (Thr180/Tyr182), and the phosphorylated MAP kinase induces caspase-11, which caspase-11 then activates caspase-1. The thus-activated caspase-1 processes IL-18 precursor to form the mature type in the adrenal, and this mature type is secreted in plasma. This pathway is referred to as "the cytokine cascade in non-inflammatory stress (stress cascade)".

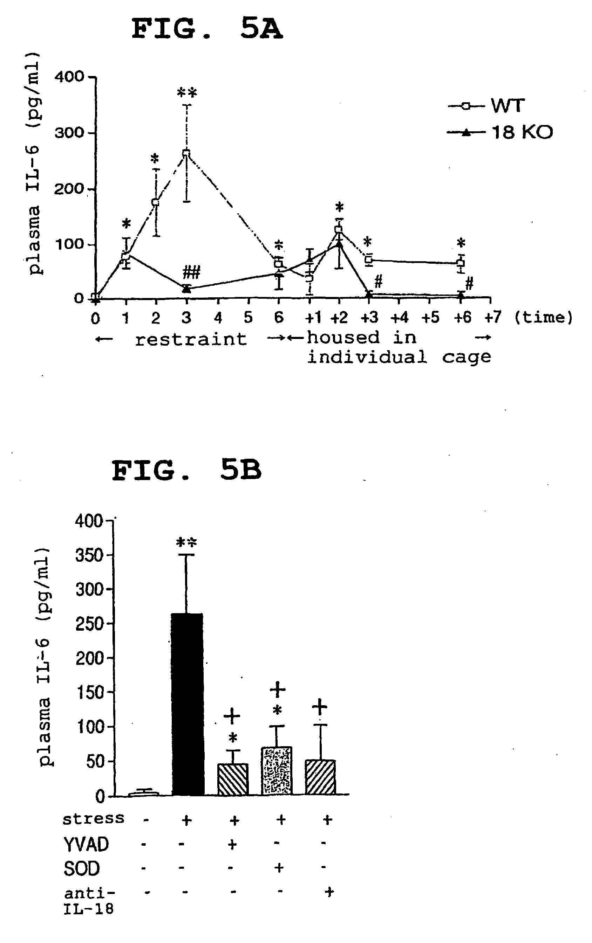

Because IL-18 activates TLR2 and Myd88, which are necessary for the induction of IL-6, the possibility that IL-18 induces IL-6 is suggested. The present inventor found that plasma IL-6 levels rose in stress-loaded wild type animals but did.not rise in IL-18 KO animals (Fig. 5A). This suggests that IL-6 may be induced IL-18-dependently. This is supported by the result that when animals were treated with a caspase-1 inhibitor and SOD, the stress-induced elevations of IL-18 and IL-6 in plasma were inhibited, and the result that in stress-loaded animals, an anti-IL-18 antibody suppressed IL-6 levels. These results represent the first evidence that non-inflammatory stress raises plasma IL-6 levels IL-18-dependently.

Accordingly, the indicator agent for non-inflammatory stress responses mediated by superoxide comprises an ingredient involved in the cytokine cascade in non-inflammatory stress. The ingredient involved in the cytokine cascade comprises IL-18 and P38MAP kinase. Although the aforementioned IL-18 occurs as the 24-kD precursor or 18-κD mature form (or also referred to as the active type) protein in humans or mice, it is preferable that the IL-18 be active type IL-18 present in blood, more preferably in plasma. The aforementioned active type IL-18 is exemplified by one having the 37th to 193rd amino acids in the amino acid sequence of SEQ ID NO:2 in the case of humans.

Judging from the aforementioned finding, the indicator agent comprises P38MAP kinase in addition to IL-18. The aforementioned P38MAP kinase is more preferably a phosphorylated one, and it is more preferable that the phosphorylation site of the aforementioned P38MAP kinase be Thr180 or Tyr182 in the case of mice, or Thr185 or Tyr187 in the case of humans.

Judging from the aforementioned finding, the indicator agent preferably comprises caspase-11 in addition to IL-18 and P38MAP kinase.

Judging from the aforementioned finding, the indicator agent preferably comprises caspase-1 in addition to IL-18, P38MAP kinase and caspase-11. Although the aforementioned caspase-1 may be procaspase-1 or activated caspase-1 (mature form), activated caspase-1 is preferable because it results in the processing of IL-18 precursor. The aforementioned activated caspase-1 is exemplified by one having the 120th to 404th amino acids in the amino acid sequence of SEQ ID NO:8 in the case of humans.

Although the indicator agent may comprise caspase-11 and/or caspase-1, as long as it comprises at least IL-18 and P38MAP kinase, the aforementioned cytokines and enzymes that constitute the indicator agent are preferably housed in separate containers. Also, the indicator agent may comprise another ingredient, as long as it has any of the aforementioned cytokines and enzymes as the major ingredient. As the other ingredient, solvents such as buffer solutions and physiological saline, stabilizers, bacteriostatic agents, antiseptics and the like can be mentioned, but are not to be construed as limiting.

The indicator agent serves as an index for the exposure and responding of an animal to non-inflammatory stress, preferably physical stress, chemical stress, mental stress and oxidative stress, more preferably stress due to ultraviolet irradiation, stress due to chemical substance exposure, restraint stress and oxidative stress caused thereby, particularly preferably to restraint stress. Particularly, by using active type IL-18 in blood, preferably in plasma, of an animal, as the index, the degree of stress can be known easily and quantitatively. The amount of IL-18 in plasma, in the case of mice, for example, is not more than 100 pg/ml before stress loading, becomes 120 to 200 pg/ml at 5 hours after 1 hour of stress loading, and exceeds 1000 pg/ml after 6 hours of stress loading; as stress loading increases, the amount of IL-18 increases. Furthermore, also because the half-life of IL-18 is about 10 hours, an indicator agent comprising IL-18 is suitable as an index for non-inflammatory stress responses. In addition to IL-18, an elevation of the phosphorylation of P38MAP kinase in the adrenal, an increase in the amount of caspase-11, or an elevation of the activity of caspase-1 also serves as an index for non-inflammatory stress responses. Specifically, it is preferable to supply a range of concentrations of IL-18 contained in the indicator agent, from a normal value to concentrations causing weak stress to strong stress, in preparation for a comparison with samples.

Also, because the indicator agent contains ingredients involved in the stress cascade, including IL-18, it can be utilized for screening of a substance that reduces these ingredients and activities. Although the screening method using the indicator agent is not subject to limitation; examples include a method comprising administering various substances to a stress-loaded animal, and selecting a substance that significantly reduces the amount of active type IL-18 in blood. Substances selected by such a screening method can be used as active ingredients for a pharmaceutical or a food with health claims, which enable the management of stress.

Each visualizing agent for non-inflammatory stress responses is intended to detect the aforementioned indicator agent. A visualizing agent for use in the invention comprises an IL-18 antibody for detecting IL-18. The IL-18 antibody is more preferably one that specifically recognizes active type IL-18.

The visualizing agent for use in the invention preferably further comprises a P38MAP kinase antibody in addition to the IL-18 antibody. The P38MAP kinase antibody is preferably an anti-phosphorylated P38MAP kinase antibody. The aforementioned anti-phosphorylated P38MAP kinase antibody more preferably recognizes a P38MAP kinase having phosphorylated Thr180 or Tyr182 in the case of mice, and recognizes a P38MAP kinase having phosphorylated Thr185 or Tyr187 in the case of humans.

The visualizing agent preferably further comprises a caspase-11 antibody in addition to the IL-18 antibody and P38MAP kinase antibody.

The visualizing agent preferably further comprises caspase-1 antibody, in addition to the IL-18 antibody, P38MAP kinase antibody and caspase-11. The aforementioned caspase-1 antibody is more preferably one that specifically recognizes activated caspase-1.

The visualizing agent preferably further comprises a substrate enabling a measurement of caspase-1 activity, in addition to the IL-18 antibody, P38MAP kinase antibody and caspase-11. The aforementioned substrate is more preferably one that emits fluorescence upon cleavage with enzyme, in order to facilitate its detection. As examples of such a substrate, Ac-YVAD-MCA, which is described in an Example, can be mentioned.

The visualizing agent preferably comprises at least an IL-18 antibody and a P38MAP kinase antibody, and may further comprise 1 kind or 2 kinds or more of caspase-11 antibody, caspase-1 antibody and caspase-1 substrate, but the aforementioned antibody or substrate that constitutes the visualizing agent is preferably housed in separate containers. Also, the visualizing agent may comprise another ingredient, as long as it has the aforementioned antibody or substrate as the major ingredient. As the other ingredient, buffer solutions, solvents such as physiological saline, stabilizers, bacteriostatic agents, antiseptics and the like can be mentioned, but are not to be construed as limitative.

The aforementioned antibody or substrate is useful as a tool (stress marker) enabling a determination of whether or not a subject is exposed to non-inflammatory stress or a measurement of the degree of the stress response thereof by detecting the presence or absence or degree of the aforementioned indicator agent in the subject.

Also, the aforementioned antibody is also useful as a tool (stress marker) for detecting a change in the expression of any of IL-18, P38MAP kinase, caspase-11 and caspase-1 in the prevention, amelioration or prediction of a change in immune status based on non-inflammatory stress.

The aforementioned antibody is not subject to limitation as to the form thereof, and may be a polyclonal antibody against any of IL-18, P38MAP kinase, caspase-11 and caspase-1 as the immune antigen, or a monoclonal antibody thereof. Furthermore, antibodies having antigen bindability to polypeptides consisting of usually at least 8 consecutive amino acids, preferably 15 amino acids, more preferably 20 amino acids, in the amino acid sequences of these proteins, are also included.

Methods of producing these antibodies are publicly known, and the aforementioned antibody can also be produced according to a conventional method (Current Protocol in Molecular Biology, Chapter 11.12 to 11.13 (2000)). Specifically, when the antibody of the present invention is a polyclonal antibody, the antibody can be obtained by using IL-18, P38MAP kinase, caspase-11 or caspase-1 expressed and purified in Escherichia coli and the like according to a conventional method, or by synthesizing an oligopeptide having a partial amino acid sequence of these proteins according to a conventional method, and immunizing a non-human animal such as a domestic rabbit therewith, and separating the antibody from the serum of the immunized animal according to a conventional method. Meanwhile, in the case of a monoclonal antibody, the antibody can be obtained hybridoma cells prepared by immunizing a non-human animal such as a mouse with IL-18, P38MAP kinase, caspase-11 or caspase-1 expressed in Escherichia coli and purified and the like according to a conventional method, or an oligopeptide having a partial amino acid sequence of these proteins, and cell-fusing the splenocytes obtained and myeloma cells (Current protocols in Molecular Biology edit. Ausubel et al. (1987) Publish. John Wiley and Sons. Section 11.4 to 11.11).

The IL-18, P38MAP kinase, caspase-11 or caspase-1 used as the immune antigen in preparing an antibody, and included in the indicator agent, can be obtained on the basis of the sequence information on the genes provided by the present invention (SEQ ID NO:1, 3, 5, 7), by operations of DNA cloning, construction of each plasmid, transfection to a host, cultivation of a transformant, and protein recovery from the culture. These operations can be performed by a method known to those skilled in the art, or in accordance with methods described in the literature (Molecular Cloning, T. Maniatis et al., CSH Laboratory (1983), DNA Cloning, DM. Glover, IRL PRESS (1985)) and the like.

Specifically, by preparing a recombinant DNA (expression vector) that allows the gene encoding IL-18, P38MAP kinase, caspase-11 or caspase-1 to be expressed in a desired host cell, introducing the same into a host cell to transform the cell, culturing the transformant, and recovering the desired protein from the culture obtained, a protein as an immune antigen for producing the antibody of the present invention can be obtained. Also, partial peptides of these IL-18, P38MAP kinase, caspase-11 or caspase-1 can also be produced by a common chemical synthesis method (peptide synthesis) according to the amino acid sequence information provided (SEQ ID NO:2, 4, 6, 8). For producing an anti-phosphorylated P38MAP kinase antibody, a peptide containing phosphorylated Thr or Tyr may be synthesized.

The method of the present invention of measuring the degree of non-inflammatory stress comprises measuring an increase in the amount of activity of IL-18 and an increase in the activity of P38MAP kinase using an IL-18 antibody and a P38MAP kinase antibody as a visualizing agent, wherein the amount or activity of IL-18 is measured in adrenal tissue or blood collected from an animal and the activity of P38MAP kinase is measured in adrenal tissue collected from an animal.

In the above-described method, the "non-inflammatory stress" is preferably physical stress, chemical stress, mental stress or oxidative stress, more preferably stress due to ultraviolet irradiation, stress due to chemical substance exposure, restraint stress, or oxidative stress caused thereby, and particularly preferably restraint stress.

Blood and tissue collected from an animal are the subject of measurement in the present method. As examples of the aforementioned animal, human, bovine, horse, swine, sheep, goat, dog, cat, mice, rats, rabbit, goat, hamster, chicken and the like can be mentioned.

In the above-described method, the detection, quantitation and the like of the indicator agent can be performed by any conventionally known method using the visualizing agent. The detection, quantitation and the like of the indicator agent can be performed using these visualizing agents according to, for example, ELISA, Western blot analysis and the like and methods based thereon. Because the aforementioned visualizing agent has the property to bind specifically to IL-18, P38MAP kinase, caspase-11 or caspase-1, the aforementioned cytokines or enzymes expressed in animal tissue can be detected specifically.

The above-described method, for example, when IL-18 is used as the index, enables an easy measurement because no more than the detection of active type IL-18 released into blood or plasma is necessary. As described above, the amount of IL-18 in plasma, in the case of mice, for example, is not more than 100 pg/ml before stress loading, becomes 120 to 200 pg/ml at 5 hours after 1 hour of stress loading, and exceeds 1000 pg/ml after 6 hours of stress loading; as stress loading increases, the amount of IL-18 also increases. Furthermore, also because the half-life of IL-18 is about 10 hours, an indicator agent comprising IL-18 is suitable as an index for non-inflammatory stress responses.

Also, in the case of caspase-11 or caspase-1, the degree of stress can also be measured by detecting the product resulting from the reaction with the aforementioned substrate. Specifically, for example, a substrate for caspase-11 or caspase-1 manipulated to produce fluorescence upon cleavage is administered to an animal, and fluorescence released into blood upon cleavage of the substrate in the adrenal can be detected. From the intensity of fluorescence detected, the degree of elevation of the aforementioned enzyme activity is known, and by comparing the elevation with a predetermined reference value, the degree of non-inflammatory stress can be measured. Alternatively, by administering to an animal the above-described antibody conjugated with a fluorescent agent, a radioactive molecule or a metal, and examining the accumulation of the fluorescence, radioactive molecule or metal in the adrenal using CT (computed tomography), MRI (magnetic resonance imaging), PET (positron emission tomography) or SPECT (single photon emission computed tomography) and the like, IL-18, P38MAP kinase, caspase-11 or caspase-1 can be detected. From the detected amount accumulated, the presence or absence or abundance of each ingredient of these stress response indicator agents is known, and by making a comparison with a predetermined reference value, the degree of stress in the sample can be measured.

According to the present invention, use of an assay kit in a method of measuring non-inflammatory stress is provided. The kit comprises the above-described visualizing agents.

These visualizing agents contained in the kit are not subject to limitation, as long as they detect IL-18 and P38MAP kinase, and optionally caspase-11 and or caspase-1. Preferably the IL-18 antibody contained in the kit is one that specifically recognizes active type IL-18. Using the kit, it is possible to measure the degree of non-inflammatory stress in the subject of measurement conveniently and quickly. Also, the assay kit can further comprise other optionally chosen reagents necessary in performing a measurement and detection. Specifically, for example, fluorescently labeled IL-18, active type I-18, assay buffer solution and the like can be mentioned as examples.

In the above-described kit, the "non-inflammatory stress" is preferably physical stress, chemical stress, mental stress or oxidative stress, more preferably stress due to ultraviolet irradiation, stress due to chemical substance exposure, restraint stress, or oxidative stress caused thereby, and particularly preferably restraint stress.

In diagnosing a change in immune status based on a non-inflammatory stress response, it is preferable to predetermine a normal value (reference value) of IL-18 in blood according to the kind of animal, to measure the amount of IL-18 in blood of the subject of diagnosis, and to compare the measured value with the normal value. If the measured value is significantly higher than the normal value (reference value), it can be judged that a change in immune status has occurred.

Because a non-inflammatory stress response mediated by superoxide has caused a change in immune status, it is preferable to further measure a peroxide in blood, spinal fluid, urine or saliva produced by superoxide in the aforementioned assay method or assay kit, or in the prophylactic, ameliorating or predicting method described below.

The aforementioned peroxide is not subject to limitation; peroxidized lipids produced upon peroxidation reactions of lipids, such as acrolein, 4-hydroxynonenal, malondialdehyde, 4-hydroxy-2-hexenal, and crotonaldehyde, peroxidated saccharides such as methylglyoxal, and oxidized bases such as 8-hydroxy-2'-deoxyguanosine can be mentioned. Confirmation of a significant elevation of peroxide and a significant elevation of the indicator agent according to the present invention is preferable because it provides a strong piece of evidence that the animal has an change in immune status.

A visualizing agent comprising an antibody enables the prevention, amelioration or prediction of a change in immune status based on a non-inflammatory stress response when applied to an animal.

The aforementioned animal is preferably a vertebral animal other than a human, and is particularly preferably a livestock or companion animal such as a bovine, horse, swine, sheep, goat, chicken, dog, or cat. By applying the prophylactic, ameliorating or predicting method to a livestock or companion animal, an effect of keeping the animal in a good immune status and rearing animals unlikely to contract disease can be obtained in the field of animal husbandry or pet industry, in which artificial rearing is likely to lead to stress loading.

As diseases that occur, exacerbate or recur due to non-inflammatory stress, appendicitis, digestive ulcers, gastric ulcer, duodenal ulcer, peritonitis, pancreatitis, ulcerative, pseudomembranous, acute and ischemic colitis, diverticulitis, epiglottitis, achalasia, cholangitis, cholecystitis, hepatitis, Crohn's disease, enterocolitis, Whipple's disease, (bronchial) asthma, allergies, anaphylactic shock, immune complex disease, ischemia of organs, reperfusion disorders, necrosis of organs, hay fever, sepsis, septicemia, endotoxin shock, cachexia, hyperpyrexia, eosinophilic granuloma, granulomatous disease, sarcoidosis, septic abortion, epididymitis, vaginitis, prostatitis, urethritis, bronchitis, pulmonary emphysema, pulmonary edema, rhinitis, cystic fibrosis, pneumonia, pneumoconiosis, alveolitis, bronchiolitis, pharyngitis, pleurisy, sinusitis, influenza, respiratory syncytial virus infection, herpes infection, HIV infection, hepatitis B virus infection, hepatitis C virus infection, disseminated bacteremia, dengue fever, candidiasis, malaria, filariasis, amebiasis, hydatid cysts, burns, dermatitis, dermatomyositis, sunburns, urticaria, warts, wheals, angiitis, vasculitis, endocarditis, arteritis, atherosclerosis, thrombophlebitis, pericarditis, myocarditis, myocardial ischemia, periarteritis nodosa, rheumatic fever, Alzheimer's disease, Alzheimer type senile dementia, diffuse Lewy body disease, Pick's disease, Binswanger's disease, Parkinson's disease, Parkinson syndrome, cerebral ischemia, cerebral ischemia/reperfusion disorders, neonatal hemorrhagic encephalopathy, epilepsy, HIV encephalopathy, brain circulatory disorders, cerebrovascular disorders, celiac disease, congestive heart failure, adult respiration distress syndrome, meningitis, encephalitis, multiple sclerosis, cerebral infarction, cerebral stroke, Guillain-Barré syndrome, neuritis, neuralgia, spinal injury, paralysis, uveitis, arthritic eruption, arthralgia, osteomyelitis, fasciitis, Paget's disease, articular rheumatism, arthritis, gout, periodontal disease, rheumatoid arthritis, synovitis, myasthenia gravis, thyroiditis, systemic lupus erythematosus (SLE), Goodpasture's syndrome, Behçet's syndrome, graft rejection in homologous transplantation, graft-versus-host disease, type I diabetes mellitus, ankylosing spondylitis, Buergur's disease, type II diabetes mellitus, ankylosing spondylitis, Buergur's disease, Reiter's syndrome, Hodgkin's disease, liver cirrhosis, renal insufficiency, allergic asthma, nephritis, myocardial infarction, Sjoegren's syndrome, contact dermatitis, atopic dermatitis, depression, eating disorders, allotriophagy, taste disorders, acne vulgaris, pemphigus, ichthyosis, psoriasis, burns, photosensitivity, ultraviolet skin disorders, wound healing, or radiation disorders, carbon monoxide poisoning, thinner poisoning, hypoxia or secondary disorders thereto, endometriosis, adrenal insufficiencies, emmeniopathy, cardiomyopathy, psychosomatic disorders, amyotrophic lateral sclerosis (ALS), oligospermia, sexual desire reduction, hypopituitarism, pituitary insufficiencies and the like can be mentioned.

As the aforementioned autoimmune disease or mental disease, appendicitis, digestive ulcers, gastric ulcer, duodenal ulcer, peritonitis, pancreatitis, ulcerative, pseudomembranous, acute and ischemic colitis, diverticulitis, epiglottitis, achalasia, cholangitis, cholecystitis, hepatitis, Crohn's disease, enterocolitis, Whipple's disease, asthma, allergies, anaphylactic shock, immune complex disease, ischemia of organs, reperfusion disorders, necrosis of organs, hay fever, sepsis, septicemia, endotoxin shock, cachexia, hyperpyrexia, eosinophilic granuloma, granulomatous disease, sarcoidosis, septic abortion, epididymitis, vaginitis, prostatitis, urethritis, bronchitis, pulmonary emphysema, rhinitis, cystic fibrosis, pneumonia, pneumoconiosis, alveolitis, bronchiolitis, pharyngitis, pleurisy, sinusitis, influenza, respiratory syncytial virus infection, herpes infection, HIV infection, hepatitis B virus infection, hepatitis C virus infection, disseminated bacteremia, dengue fever, candidiasis, malaria, filariasis, amebiasis, hydatid cysts, burns, dermatitis, dermatomyositis, sunburns, urticaria, warts, wheals, angiitis, vasculitis, endocarditis, arteritis, atherosclerosis, thrombophlebitis, pericarditis, myocarditis, myocardial ischemia, periarteritis nodosa, rheumatic fever, Alzheimer's disease, celiac disease, congestive heart failure, adult respiration distress syndrome, meningitis, encephalitis, multiple sclerosis, cerebral infarction, cerebral stroke, Guillain-Barré syndrome, neuritis, neuralgia, spinal injury, paralysis, uveitis, arthritic eruption, arthralgia, osteomyelitis, fasciitis, Paget's disease, gout, periodontal disease, rheumatoid arthritis, synovitis, myasthenia gravis, thyroiditis, systemic lupus erythematosus, Goodpasture's syndrome, Behçet's syndrome, graft rejection in homologous transplantation, graft-versus-host disease, type I diabetes mellitus, ankylosing spondylitis, Buergur's disease, type II diabetes mellitus, ankylosing spondylitis, Buergur's disease, Reiter's syndrome, Hodgkin's disease, SLE, liver cirrhosis, renal insufficiency, allergic asthma, nephritis, myocardial infarction, Sjoegren's syndrome, contact dermatitis, atopic dermatitis, depression, eating disorders, allotriophagy, taste disorders, acne vulgaris, pemphigus, ichthyosis, psoriasis, burns, photosensitivity, ultraviolet skin disorders, or radiation disorders, carbon monoxide poisoning, hypoxia or secondary disorders thereto and the like can be mentioned.

As the malignant tumors, hematopoietic organ tumors such as acute leukemia, chronic leukemia, malignant lymphoma, multiple myeloma, and macroglobulinemia, as well as solid tumors such as colorectal cancer, brain tumors, head-and-neck cancer, breast cancer, lung cancer, esophageal cancer, gastric cancer, liver cancer, gall bladder cancer, cholangiocarcinoma, pancreas cancer, islet cell carcinoma, renal cell carcinoma, adrenal cortex cancer, urinary bladder cancer, prostatic cancer, testicular tumor, ovarian cancer, uterine cancer, choriocarcinoma, thyroid cancer, malignant cartinoid tumor, skin cancer, malignant melanoma, osteosarcoma, soft tissue sarcoma, neuroblastoma, Wilms tumor, and retinoblastoma, can be mentioned.

An elevation of IL-18 in blood is followed by the release of various cytokines. In particular, IL-6 is involved in many inflammatory diseases and is drawing attention as an exacerbation factor. As diseases known to be caused or exacerbated due to a systemic elevation of these cytokines, out of the above-described diseases, diseases involved by inflammatory mechanisms, such as appendicitis, peritonitis, pancreatitis, ulcerative, pseudomembranous, acute and ischemic colitis, diverticulitis, epiglottitis, cholangitis, cholecystitis, hepatitis, epididymitis, vaginitis, prostatitis, urethritis, bronchitis, pneumonia, pneumoconiosis, alveolitis, bronchiolitis, pharyngitis, pleurisy, sinusitis, dermatitis, dermatomyositis, angiitis, vasculitis, endocarditis, arteritis, atherosclerosis, thrombophlebitis, pericarditis, myocarditis, myocardial ischemia, periarteritis nodosa, osteomyelitis, fasciitis, rheumatic fever, meningitis, encephalitis, Paget's disease, gout, periodontal disease, thyroiditis, neuritis, uveitis, type I diabetes mellitus, gastric ulcer, digestive ulcers, and duodenal ulcer; diseases known to be involved by cytokines or autoimmune mechanisms, such as Crohn's disease, ulcerative colitis, Sjoegren's syndrome, multiple sclerosis, Guillain-Barré syndrome, dermatomyositis, systemic lupus erythematosus, bronchial asthma, immune complex disease, eosinophilic granuloma, granulomatous disease, sarcoidosis, articular rheumatism, periarteritis nodosa, ankylosing spondylitis, Behçet's syndrome, graft rejection in homologous transplantation, graft-versus-host disease, malignant hyperpyrexia, habitual abortion, and endometriosis; infectious diseases such as hay fever, influenza, respiratory syncytial virus infection, herpes infection, HIV infection, hepatitis B virus infection, hepatitis C virus infection, disseminated bacteremia, dengue fever, candidiasis, malaria, filariasis, amebiasis, and hydatid cysts; and diseases that have not completely been clarified pathologically but are shown to be involved by the above-described cytokines, such as achalasia, Whipple's disease, burns, sunburns, urticaria, warts, wheals, angiitis, vasculitis, endocarditis, arteritis, Alzheimer's disease, celiac disease, congestive heart failure, adult respiration distress syndrome, cerebral infarction, cerebral stroke, gout, myasthenia gravis, amyotrophic lateral sclerosis, thyroiditis, Buergur's disease, type II diabetes mellitus, obesity, Reiter's syndrome, Hodgkin's disease, liver cirrhosis, renal insufficiency, myocardial infarction, Sjoegren's syndrome, depression, eating disorders, allotriophagy, and taste disorders; and the like can be mentioned.

As other diseases and conditions that occur, exacerbate or recur with the involvement of IL-18 and cytokines released thereby, circadian rhythm abnormalities, appetite abnormalities, pituitary insufficiencies such as Addison's disease, acromegaly, and gonadal developmental disorders, which are abnormalities of the control of the nervous and endocrine systems; thyroid functional impairment, obesity, glucose control abnormalities; the secondary occurrence, recurrence or exacerbation of primary aldosteronism, Cushing's disease, and diseases pathologically characterized by joint inflammation, such as articular rheumatism, gout and arthritis, due to non-inflammatory stress such as cold or mental stress, insufficient sleep, bloodstream disorders, physical weight loading and the like; renal tubular epithelial cell loss due to infection, poisons, and anticancer agents that injure the renal tubules, such as cisplatin and carboplatin, or by an idiopathic mechanism, can be mentioned.

Furthermore, according to the present invention, provided is a method of screening for a substance having a therapeutic effect, ameliorating effect or prophylactic effect on a disease that occurs, exacerbates or recurs due to mental stress, or a substance having a suppressive effect or preventive effect on oxidative stress, which comprises measuring the amount or activity of IL-18 and the activity of P38MAP kinase. According to the method of the present invention, screening for a substance having a therapeutic, ameliorating or prophylactic effect on a disease involved directly or indirectly by mental stress can be performed in large amounts, quickly, and conveniently. A substance screened for by this method is effective as a therapeutic drug, ameliorating drug or prophylactic drug for a disease that occurs, exacerbates or recurs due to mental stress. Also, according to the present screening method, a substance having a suppressive effect or preventive effect on oxidative stress can be screened for. Because superoxide is present upstream of the cytokine cascade in non-inflammatory stress, according to the present screening method, a substance having a suppressive effect or preventive effect on oxidative stress can be screened for with the aforementioned indicator agent as the index, and the present screening method is helpful in developing pharmaceuticals and foods with health claims (foods for specified health uses and foods with nutrient function claims) with such a substance as an active ingredient.

The screening method of the present invention comprises, specifically, for example, the following steps:
1) rearing an animal under test substance administration conditions,
2) quantifying the amount or activity of IL-18 in adrenal tissue or blood collected from the animal and the activity of P38MAP kinase in adrenal tissue collected from the animal, and
3) comparing the amount or activity of the IL-18 and the activity of the P38MAP kinase in the presence and absence of test substance, and judging that a test substance that has significantly reduced or increased the amount or activity of IL-18 and the amount of P38MAP kinase is a substance having a therapeutic effect, ameliorating effect or prophylactic effect on a disease that occurs, exacerbates or recurs due to mental stress, or is a substance having a suppressive effect or preventive effect on oxidative stress.

As examples of animals used in the present screening method, mouse, rat, rabbit, goat, sheep, hamster, chicken and the like can be mentioned, with preference given to mouse and rat because of the ease of operation. Also, a model animal or transgenic animal for a disease that occurs, exacerbates or recurs due to mental stress may be used.

Also, the animal used may or may not be previously loaded with non-inflammatory stress. For example, when a substance having a therapeutic effect and ameliorating effect on a disease that occurs, exacerbates or recurs due to mental stress is screened for, the animal may be previously loaded with non-inflammatory stress, and thereafter given a test substance, and the like. When a substance having a prophylactic effect on the aforementioned disease is screened for, a test substance may be administered and the like before the animal is loaded with mental stress.

As examples of the aforementioned test substance, peptides, proteins, synthetic compounds, extracts from cell or plant and the like can be mentioned, and these may be novel substances or known substances. As examples of the test substance when a substance having a suppressive effect or preventive effect on oxidative stress is screened for, various vitamins, thiol compounds, various plant-derived polyphenols, antioxidant peptides/amino acids, probucol, which is used as a therapeutic drug for hyperlipemia, captopril, which is used as a hypotensive agent, and the aforementioned superoxide suppressant can be mentioned.

As the sample prepared from an animal, blood and tissue collected from an animal are used.

As the aforementioned IL-18 indicator agent, active type IL-18 is particularly preferable. Although the indicator agent in the sample can be quantified by any conventionally known method, it is preferable to use a visualizing agent; using these visualizing agents, this quantitation can be performed according to, for example, ELISA, Western blot analysis and the like and methods based thereon.

The present screening method can be suitably used for screening for a substance having a therapeutic effect, ameliorating effect or prophylactic effect preferably on systemic lupus erythematosus, lupus nephritis, atopic dermatitis, psoriasis, cerebral ischemia, cerebral degeneration, encephalitis, articular rheumatism, multiple sclerosis, bronchial asthma, cardiomyopathy, emmeniopathy, endometriosis, and sexual desire reduction, and particularly preferably on lupus nephritis.

The present screening method can be performed by rearing an animal in a stressed state under test substance administration conditions and, to the extent the method involves IL-18, measuring the concentration of IL-18 contained in the blood of the aforementioned animal, comparing this with the blood concentration of IL-18 obtained when the animal is reared under test substance non-administration conditions, and judging a test substance as described above that has significantly reduced or increased the blood concentration of IL-18 to be a substance having a therapeutic effect, ameliorating effect or prophylactic effect on a disease that occurs, exacerbates or recurs due to the stress.

### Examples

The present invention is hereinafter described in more detail by means of the following Examples.

### [Animals]

Male C57B6 mice were purchased from Seac Yoshitomi. IL-18 knockout (KO) mice (C57/B6 background, Immunity. 8, 383-390 (1998)) and caspase-1 knockout (KO) mice (Balb/C background, Science. 267, 2000-2003 (1995)) were supplied by Dr. Takeda and Dr. Kuida, respectively. MRL/MpJUmmCrj-lpr/lpr (MRL/lpr) mice were purchased from Charles River Japan Inc. The mice were housed at 20°C at 1 animal per cage. Experiments using the mice were started at 10 o'clock.

### [Reagents]

An anti-ACTH antiserum manufactured by Progen Biotechnik (Germany), an anti-mouse IL-18 monoclonal antibody manufactured by MBL (Japan), an anti-mouse caspase-1 antibody (sc-515) manufactured by Santa Cruz (USA), an anti-mouse/rat caspase-11 antibody manufactured by BIOMOL (USA), and an anti-phosphorylated (Thr180/Tyr182) P38 MAP kinase antibody manufactured by Cell Signaling Technology (USA) were used. An anti-mouse IL-6 polyclonal antibody manufactured by Chemi-Con Company was used. For an anti-mouse IL-18 receptor antibody, a partial peptide (20 amino acids) of the hydrophilic region in the immediate vicinity of the transmembrane region of mouse IL-18 receptor was synthesized according to a conventional method, and immunized to a rabbit, and the antiserum obtained was used.

Synthetic ACTH (1-24 amino acids) was purchased from Peninsula Laboratories Inc. (USA). Ac-YVAD-CHO and Ac-YVAD-MCA were purchased from Peptide Institute, Inc. Z-FA-fmk (J. Biol. Chem. 276, 21153-21157 (2000)) and SB203580 were purchased from Calbiochem-Novabiochem (USA) and BIOMOL Research Laboratories, respectively. Diphenyleneiodonium chloride (DPI) and nicotinamidoadenine dinucleotide phosphate (NADPH) oxidase inhibitors and superoxide dismutase (SOD)-1 were purchased from Sigma (USA).

### [Restraint stress].

For restraint stress, each mouse was kept in a restrainer (a round plastic tube 27 mm in diameter) for 1, 3 or 6 hours, and IL-18 levels were measured. Regarding the analysis of IL-6, each mouse was restrained as described above, and then housed in the starting cage for 6 hours, after which a measurement was performed.

### [Assay for cytokines]

After restraint, each mouse was anesthetized with diethyl ether, and blood was drawn into an EDTA-containing tube. Plasma was separated by centrifugation at 4°C and 10000 x g for 10 minutes, and stored at -80°C. IL-18 and IL-6 were analyzed by ELISA using QuantikineTM immunoassay kit (R&D Systems) and OptEIATM (BD-Pharmingen), respectively.

### [Statistical analysis]

All experimental data are shown as the mean±S.D. for three or more independent experiments. Statistical comparisons among different treatments were performed by Student's t-test using the GraphPad Prism program (GraphPad Software Inc.). A P-value less than 0.05 was considered to indicate statistical significance. The symbols shown in the figures are as follows:
*; P<0.05 versus control
**; P<0.01 versus control
***; P<0.001 versus control
#; P<0.01 (n=4-9) versus stress loading
   P<0.05 (n=4-9) versus stress loading (in case of Fig. 4)
   P<0.05 versus wild type (in case of Fig. 5)
##; P<0.001 versus stress loading (n=4-9)
   P<0.01 (n=4-9) versus stress loading (in case of Fig. 4)
   P<0.01 versus wild type (in case of Fig. 5)
+; P<0.01 versus stress-loaded wild type (n=6).

### [Example 1]

### Measurement of plasma IL-18 protein levels in restraint stress mice

The anti-ACTH antiserum (50 µl, 1:1), dexamethasone (1 ng/50 µl PBS(-)) or the anti-IL-18 antibody (50 µl, 1:1) was administered intraperitoneally to each mouse; after 10 minutes elapsed, the animal was kept in a restraint tube for 6 hours. Synthetic ACTH (250 µg/50 µl PBS(-)) was injected to the left leg; after 6 hours elapsed, the mice were subjected to the experiment. The results are shown in Fig. 1.

### (1) Elevation of plasma ACTH level precedes an increase in plasma IL-18 level

From Fig. 1A, the plasma IL-18 levels in the mice exposed to restraint stress were 43±34.3 pg/ml (before stress loading), 330±64.3 pg/ml (3 hours after stress loading) and 1059±30.022 pg/ml (6 hours after stress loading). ACTH in plasma increased more rapidly than IL-18, and reached about 500 pg/ml within 1 hour after stress loading but decreased to the baseline level in 3 hours. From this fact, it is found that the elevation of plasma ACTH level precedes an increase in plasma IL-18 level. Note that an elevation of plasma ACTH level indicates the activation of the hypothalamus-pituitary-adrenal (HPA) axis.

### (2) ACTH is involved in stress-induced elevation of plasma IL-18

ACTH is known to cause the adrenal-specific expression of IL-18 mRNA. Hence, whether or not ACTH is involved in the stress-induced elevation of plasma IL-18 was examined. Mice were treated with the anti-ACTH antiserum or dexamethasone, and IL-18 levels in plasma or in the adrenal were analyzed. From Fig. 1B and Fig. 1C, it was found that the anti-ACTH antiserum and dexamethasone inhibited the stress-induced increases in IL-18 levels in plasma (Fig. 1B) and in the adrenal (Fig. 1C). In mice having one adrenal extirpated, the IL-18 levels after stress loading were lower than those in sham-operated mice. From these results, it was suggested that by stress-induced ACTH, elevations of IL-18 levels in plasma and the adrenal might be caused.

### (3) ACTH first influences IL-18 protein levels in the adrenal

When ACTH was administered, the plasma IL-18 level rose, but its degree was lower than that with stress loading (Fig. 1D). The IL-18 level in the adrenal rose to the same extent as in the case of stress loading (Fig. 1E). From these results, it is suggested that in the stress-loaded mice, ACTH mainly results in a stress-induced elevation of IL-18 in the adrenal, and that another factor might be involved in the elevation of plasma IL-18 level.

### (4) Properties of IL-18 protein

As a result of an analysis of IL-18 protein by Western blotting, it was found that IL-18 occurred as a 18-kD mature type protein in plasma (Fig. 1F, lanes 1 to 3), and on the other hand occurred mainly as a 24-kD precursor type in the adrenal (Fig. 1F, lanes 4 to 6). From these results, it is suggested that the IL-18 precursor in the adrenal may be processed and released into plasma.

### [Example 2]

### Role of caspase-1 in stress

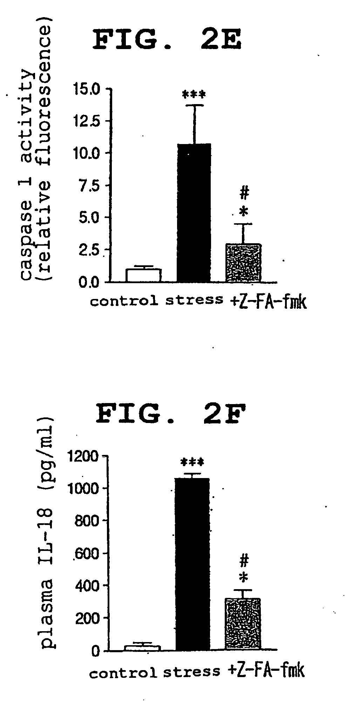

Each mouse was kept in a restrainer for 1 hour, and Ac-YVAD-CHO (200 µM/50 µl PBS(-)), Z-FA-fmk (100 µM/50 µl PBS(-)) and SB-203580 (100 µg/50 µl PBS(-) per mouse) were administered intraperitoneally. 1 and 3 hours after the start of restraint, SOD-1 (20 U) and DPI (30 µg) were administered intraperitoneally to the mouse, and samples were analyzed by Western blotting. The results are shown in Fig. 2.

Caspase-1 was induced in the adrenal by ACTH (Fig. 2A, lane 2) and stress (Fig. 2A, lane 3). Caspase-1 activity in the adrenal increased 10 fold after stress loading (Fig. 2B). The caspase-1 inhibitor YVAD-CHO effectively inhibited the stress-induced increase in plasma IL-18 level (Fig. 2C), but did not inhibit the IL-18 precursor protein level in the adrenal (Fig. 2D). In caspase-1 knockout (KO) mice, IL-18 was not detected in plasma just before and after stress loading, but after stress loading, the IL-18 precursor level increased. From these results, it is suggested that caspase-1 may be essential to the processing of the IL-18 precursor in the adrenal, and essential to the elevation of plasma IL-18 in stress-loaded mice.

### [Example 3]

### Analysis of caspase-11 and MAP kinase by Western blotting

Western blot analysis of IL-18 and phosphorylated P38MAP kinase in adrenal tissue was performed as described below. The adrenal tissue was homogenized in an immunoprecipitation (IP) buffer solution (50 mM Tris pH 7.4, 150 mM NaCl, 0.5% NP-40 and 0.5% sodium deoxycholate) containing a protease inhibitor mixture (manufactured by Roche Diagnostics) using a Polytron homogenizer. The homogenate obtained (100 µg protein) was pre-clarified using protein A/G agarose (Exalpha Biologicals, Inc.), incubated with the anti-mouse IL-18 monoclonal antibody or the anti-phosphorylated (Thr180/Tyr182) P38 MAP kinase antibody and protein A/G agarose (50 µl), and centrifuged (at 13000 x g for 25 minutes, 4°C). The precipitate was boiled, and the adrenal protein was recovered, electrophoresed in 10-20% SDS-polyacrylamide gradient gel, and electrically transferred to a Hybond ECL (manufactured by Amersham) nitrocellulose membrane. The aforementioned membrane was incubated with an antibody (IL-18, phosphorylated (Thr180/Tyr182) P38 MAP kinase, caspase-1 or caspase-11), and the reacted protein was detected using an ECL plus system (manufactured by Amersham biosciences). Procaspase-11 and activated P38 MAP kinase were quantified using a Science Lab 99 image analysis system (manufactured by Fujifilm).

### (1) Involvement of caspase-11 in the upregulation of caspase-1 activity

Since caspase-1 is reported to be activated by caspase-11, the effect of the caspase-11 inhibitor Z-FA-fmk on plasma IL-18 level was examined. The results are shown in Figs. 2E and 2F.

It was found that Z-FA-fmk suppressed the stress-induced increase in caspase-1 activity in the adrenal (Fig. 2E), and suppressed plasma IL-18 level (Fig. 2F). From this fact, it is suggested that caspase-11 may be involved in the stress-induced activation of caspase-1 in the adrenal.

### (2) Involvement of P38MAP kinase in the upregulation of caspase-1 activity

It has been reported that the induction of caspase-11 by LPS requires the activation of NF-κB, and that the activation of NF-κB is mediated by P38MAP kinase. In vitro, it has been reported that the induction of caspase-11 is suppressed by the P38MAP kinase inhibitor SB203580. In the present invention, in vivo, that is, in stress-loaded mice, the effect of SB203580 on the induction of caspase-11 was examined. The results are shown in Fig. 3.

SB203580 inhibited the stress-induced increase in procaspase-11 protein (Fig. 3A). In the SB203580-treated mice, caspase-1 activity in the adrenal (Fig. 3B) and plasma IL-18 level were also lower than those in control mice (Fig. 3C). With the treatment using SB203580 and Z-FA-fmk in combination, no further reduction in the activity of caspase-1 (Fig. 3B), plasma IL-18 level (Fig. 3C) and 24-kD IL-18 precursor level in the adrenal (not illustrated) did not occur. Neither YVAD-CHO nor Z-FA-fmk influenced the phosphorylation of P38MAP kinase in the adrenal (Fig. 3D). The phosphorylation of P38MAP kinase, like in wild type mice (Fig. 3D, lane 2), was induced by stress in caspase-1 KO mice (Fig. 3D, lane 5). Therefore, this phosphorylation did not depend on caspase-1.

### [Example 4]

### Localization of IL-18, caspase-1, caspase-11 and P38MAP kinase by immunohistochemistry

The adrenal was fixed in formalin, embedded in paraffin, and cut into 4-µm sections. The aforementioned tissue sections were incubated with an antibody (P38 MAP kinase, caspase-11, caspase-1 (these three were diluted 200 fold) or IL-18 (diluted 400 fold)). Using a horseradish peroxidase-conjugated secondary antibody (manufactured by Santa Cruz) and diaminobenzidin (DBA), color development was performed. For control, sections without the primary antibody and sections derived from a mouse without stress were used. The results are shown in Figs. 3E and 3F.

In the stress-loaded mice, IL-18, caspase-1, caspase-11 and activated P38MAP kinase were co-localized in the zona reticularis of adrenal cortex (Fig. 3E), whereas in the control mice, none of them was detected. An inhibitor of caspase-1 (Fig. 3F, panel 1) and an inhibitor of caspase-11 (Fig. 3F, panel 2) did not influence the accumulation of phosphorylated P38MAP kinase in adrenal cortex.

From the results of Example 3(2) and Example 4 above, it is shown that P38MAP kinase is involved in the stress-induced elevation of plasma IL-18 level via the induction of caspase-11, which in turn activates caspase-1.

### [Example 5]

### Analysis of caspase-1 activity

Caspase-1 activity in the adrenal was measured using a caspase assay kit (manufactured by BD-Pharmingen). Describing briefly, a homogenate of adrenal tissue was incubated with Ac-YVAD-MCA (acetyl-L-tyrosyl- L-valyl- L-alanyl- L-aspartic acid 4-methyl-coumaryl-7-amide), which is a fluorescent substrate for caspase-1, and the MCA leaving from the aforementioned substrate was measured using a fluorescence plate reader (excitation wavelength 380 nm, irradiation wavelength 420-460 nm).

### [Example 6]

### Involvement of superoxide in the upregulation of P38MAP kinase

Active oxygen species are reported to mediate the phosphorylation of P38MAP kinase in pulmonary fibroblasts, neutrophils and monocytes. In stress-loaded mice, the effect of SOD on the phosphorylation of P38MAP kinase was examined. The results are shown in Fig. 4.

It is found that SOD inhibits the phosphorylation of P38MAP kinase by about 70% in the adrenal (Fig. 4A). Also, SOD, in the stress-loaded mice, also reduced the levels of procaspase-11 (Fig. 4B), caspase-1 activity (Fig. 4C) and plasma IL-18 (Fig. 4D), but did not reduce the adrenal level of IL-18 precursor protein (Fig. 4E). Treatment with SOD in combination with SB203580, Z-FA-fmk or YVAD-CHO did not result in a further reduction in the level of procaspase-11, caspase-1 or plasma IL-18, respectively. The induction of plasma IL-18 by stress was suppressed by DPI, that is, an NADPH oxidase inhibitor (Fig. 4F). This supports the notation that a superoxide anion is involved in the induction of plasma IL-18 by stress via the phosphorylation of P38MAP kinase.

### [Example 7]

### Involvement of IL-18 in the control of plasma IL-6 level in stress-loaded mice

Using IL-18-deficient mice, the relationship between IL-6, which is another stress-related cytokine, and IL-18, was examined. Without stress, IL-6 was undetectable in WT and IL-18 KO, but after 3 hours of stress loading, the IL-6 level was 230 pg/ml in WT and 25 pg/ml in IL-18 KO (Fig. 5A). After liberation from the stress, plasma IL-6 was kept at about 80 pg/ml in WT, but decreased to 5 pg/ml in IL-18 KO (Fig. 5A). Treatment with YVAD-CHO, SOD and anti-IL-18 antibody 70% inhibited the stress-induced increase in plasma IL-6 level in WT (Fig. 5B). From these results, it is suggested that stress may induce IL-6 in plasma IL-18-dependently.

### [Example 8]

### Treatment of lupus nephritis by administration of IL-18 receptor antibody

MRL/lpr mice at 10 weeks of age were loaded with 3 hours of restraint stress, and concentrations of IL-6 in plasma were examined. Also, 10 minutes before stress loading, the anti-IL-18 receptor antibody was administered to the aforementioned mice, and changes in the concentration of IL-6 were examined. As a result, at 6 hours after stress loading, the concentration of IL-18 in plasma rose to 1132, 882 and 793 pg/ml, and the elevation of the concentration of IL-6 in plasma reached a peak 3 hours after stress loading. In the MRL/lpr mice after stress loading, an elevation of IL-6 in plasma was observed, but in the mice not loaded with stress, IL-6 was not detected. When mice receiving the anti-IL-18 receptor antibody were loaded with stress, the elevation of IL-6 was suppressed.

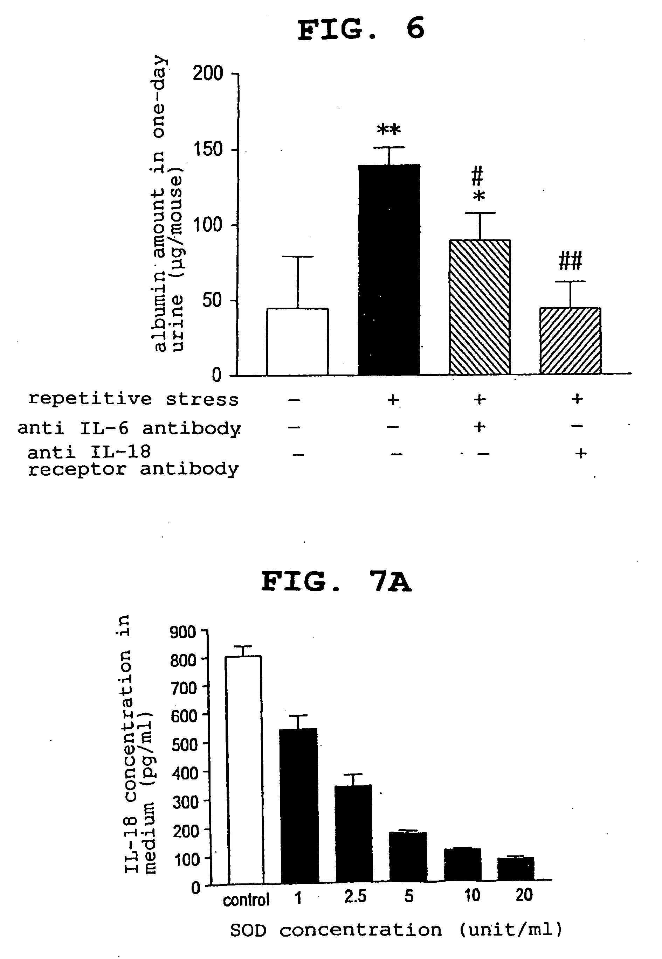

MRL/lpr mice have spontaneously developing lupus nephritis. MRL/lpr mice at 12 weeks of age were loaded with 6 hours of restraint stress every 2 days (3 times/week) for 3 weeks. The aforementioned mice were divided into a group with stress only, a group receiving the anti-IL-6 antibody, and a group receiving the anti-IL-18 receptor antibody, and albumin contents in one-day pools of urine (proteinuria) were examined. For control, MRL/lpr mice not loaded with stress were also examined. The results are shown in Fig. 6.

From Fig. 6, the MRL/lpr mice experienced increased proteinuria and exacerbated lupus nephritis when loaded with stress. However, it is found that by administering the anti-IL-6 antibody or the anti-IL-18 receptor antibody, the MRL/lpr mice have the stress-induced exacerbation of lupus nephritis suppressed. Particularly, administration of the anti-IL-18 receptor antibody was effective.

### [Example 9]

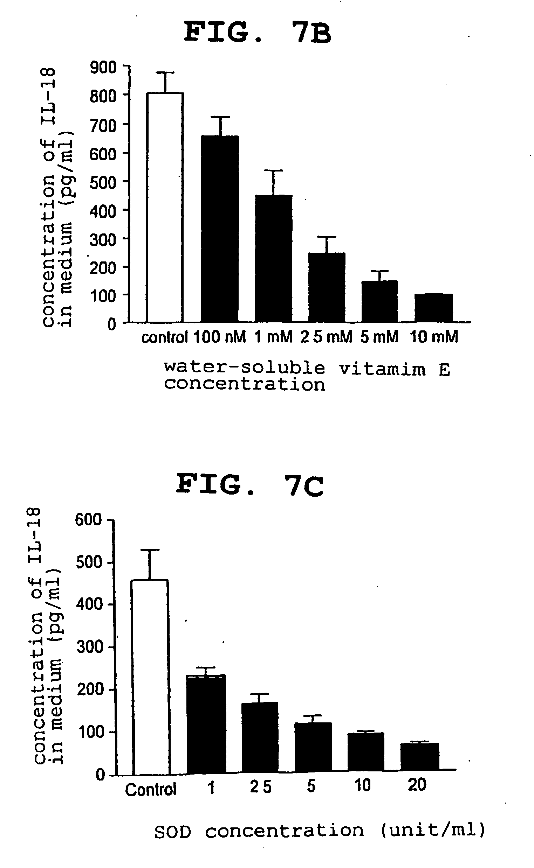

Primary culture human skin keratinocytes (keratinocytes) were cultured in a basal medium for keratinocytes contained in petri dishes for cell culture 6 cm in diameter at 1.0-2.0 x 10⁶/dish. This was cultured in the aforementioned medium containing 1 µM of paraquat, which is an active oxygen generator, for 15 minutes, after which the medium was exchanged with a paraquat-free medium, and the cells were further cultured for 1 hour, after which the medium was collected, and the concentration of IL-18 protein in the medium was measured. Also, during paraquat treatment, the cells were cultured in a medium containing SOD (1, 2.5, 5, 10, 20 U/ml), which is an active oxygen eliminator, or water-soluble vitamin E (100 nM, 1, 2.5, 5,10 mM/ml), and the concentration of IL-18 protein in the medium was measured. In the keratinocytes, the activation and release of IL-18 by the active oxygen molecular species were observed. Furthermore, these were suppressed by SOD, which is an active oxygen eliminator, or by vitamin E, which neutralizes active oxygen, dose-dependently (Figs. 7A and 7B).

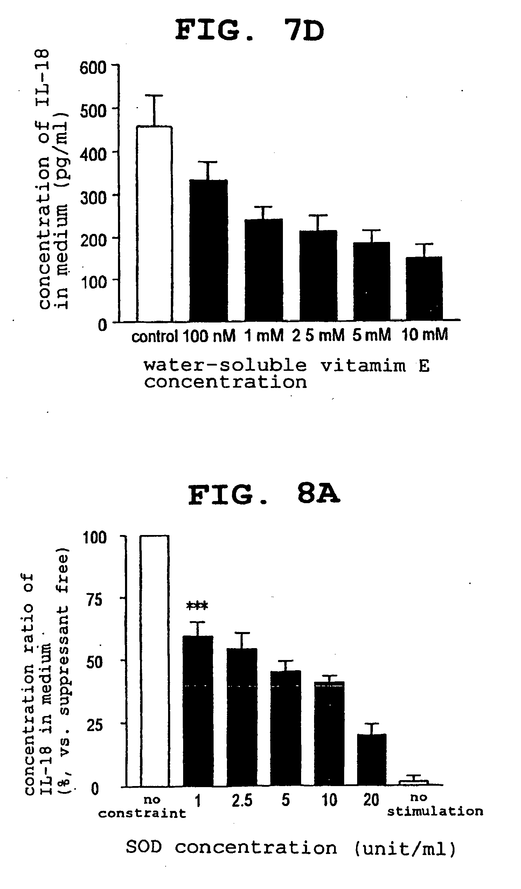

Primary culture human skin keratinocytes (keratinocytes: purchased from CAMBREX Company) were cultured in a basal medium for keratinocytes contained in petri dishes for cell culture 6 cm in diameter at 1.0-2.0 × 10⁶/dish. This was irradiated with 100 mJ of ultraviolet B rays (UV-B rays), after which the cells were cultured for 1 hour, after which the medium was collected, and the concentration of IL-18 protein in the medium was measured. Also, after the UV-B ray treatment, the cells were cultured in a medium containing SOD (1, 2.5, 5, 10, 20 U/ml), which is an active oxygen eliminator, or water-soluble vitamin E (100 nM, 1, 2.5, 5, 10 mM/ml), and the concentration of IL-18 protein in the medium was measured. In the keratinocytes, the activation and release of IL-18 by the active oxygen molecular species were observed. Furthermore, these were suppressed by SOD, which is an active oxygen eliminator, or by vitamin E, which neutralizes active oxygen, dose-dependently (Figs. 7C and 7D).

### [Example 11]

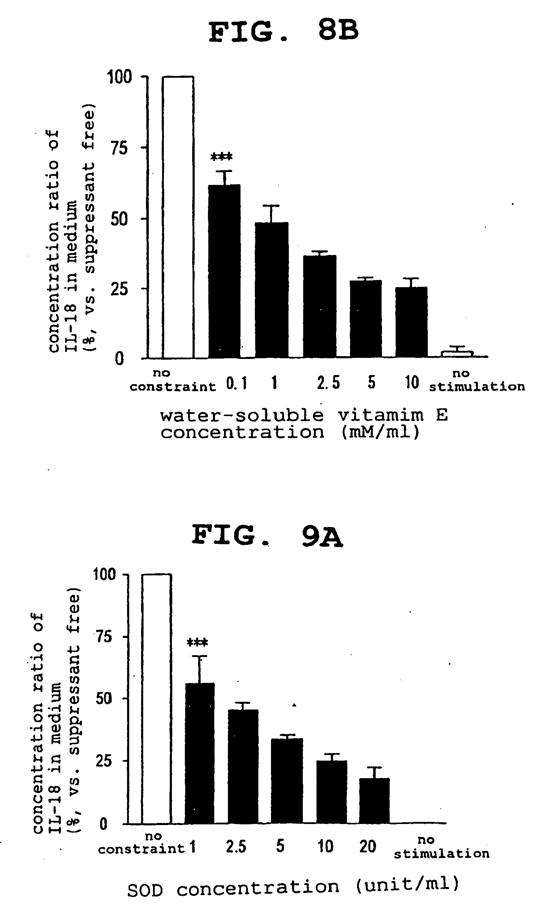

Brain microglia isolated from the mouse neonatal brain by the method of Hassan et al. (Hassan NF, Rifat S, Campbell DE, McCawley LJ, Douglas SD. J. Leukoc Biol. 1991 Jul; 50(1):86-92) were cultured at 1.5-2.0 x 10⁶/ml. This was cultured in Dulbecco's minimum essential medium containing 100 nM of paraquat, which is an active oxygen generator, for 1 hour, after which the medium was exchanged with a paraquat-free medium. After further cultivation for 3 hours, the medium was collected, and the concentration of IL-18 protein in the medium was measured. Also, after the paraquat treatment, the cells were cultured in a medium containing SOD (1, 2.5, 5, 10, 20 U/ml), which is an active oxygen eliminator, or water-soluble vitamin E (100 nM, 1, 2.5, 5, 10 mM/ml), and the concentration of IL-18 protein in the medium was measured. In the microglia, the activation and release of IL-18 by the active oxygen molecular species were observed. Furthermore, these were suppressed by SOD, which is an active oxygen eliminator, or by vitamin E, which neutralizes active oxygen, dose-dependently (Figs. 8A and 8B).

### [Example 12]

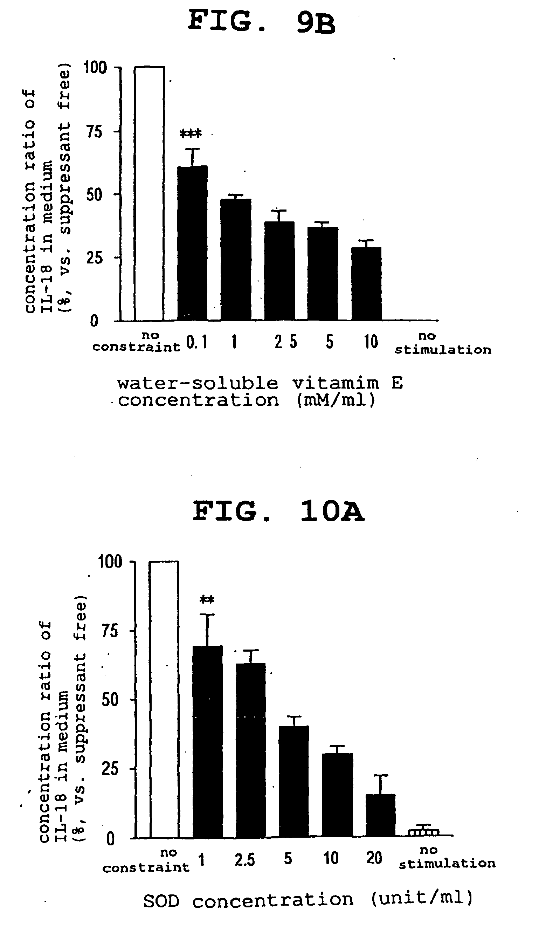

Brain astrocytes isolated from the mouse neonatal brain by the method of Yang et al. (Yang P, Hernandez MR. Brain Res Brain Res Protoc. 2003 Oct; 12(2):67-76) were prepared at 1.5-2.0 x 10⁶/ml. This was cultured in Dulbecco's minimum essential medium containing 100 nM of paraquat, which is an active oxygen generator, for 1 hour, after which the medium was exchanged with a paraquat-free medium. The cells were further cultured for 3 hours, after which the medium was collected, and the concentration of IL-18 protein in the medium was measured. Also, after the paraquat treatment, the cells were cultured in a medium containing SOD (1, 2.5, 5, 10, 20 U/ml), which is an active oxygen eliminator, or water-soluble vitamin E (100 nM, 1, 2.5, 5, 10 mM/ml), and the concentration of IL-18 protein in the medium was measured. In the astrocytes, the activation and release of IL-18 by the active oxygen molecular species were observed. Furthermore, these were suppressed by SOD, which is an active oxygen eliminator, or by vitamin E, which neutralizes active oxygen, dose-dependently (Figs. 9A and 9B).

### [Example 13]

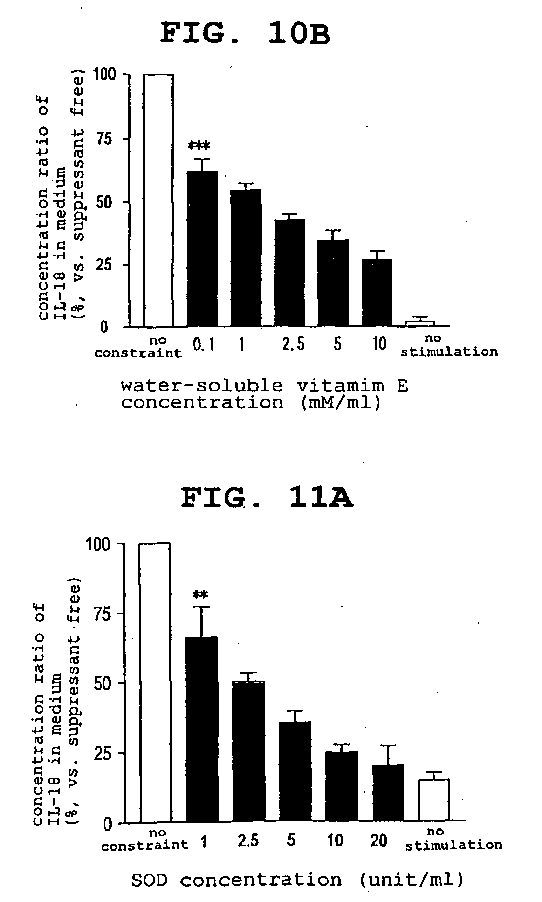

Abdominal macrophages isolated from mice by the method of Hamilton et al. (Hamilton TA, Weiel JE, Adams DO., J Immunol. 1984 May; 132(5):2285-90) were cultured in Dulbecco's minimum essential medium at 1.0-2.0 x 10⁵/dish. The medium was exchanged with the aforementioned medium containing 100 nM of paraquat, which is an active oxygen generator, and the cells were cultured for 15 minutes, after which the medium was exchanged with a paraquat-free medium, and the cells were further cultured for 3 hours, after which the medium was collected, and the concentration of IL-18 protein in the medium was measured. Also, during paraquat treatment, the cells were cultured in a medium containing SOD (1, 2.5, 5, 10, 20 U/ml), which is an active oxygen eliminator, and the concentration of IL-18 protein in the medium was measured. In the macrophages, the activation and release of IL-18 by the active oxygen molecular species were observed. Furthermore, these were suppressed by SOD, which is an active oxygen eliminator, or by vitamin E, which neutralizes active oxygen, dose-dependently (Figs. 10A and 10B).

### [Example 14]

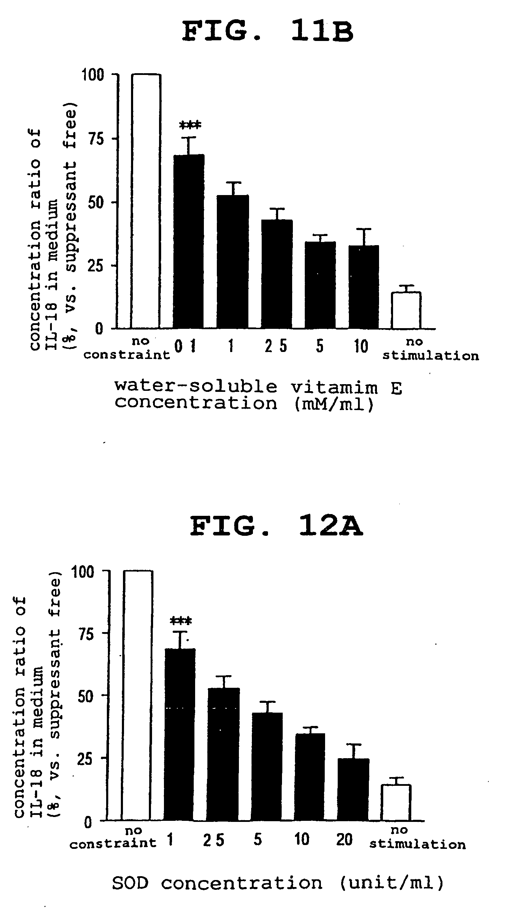

Synovial membrane tissue collected from the articular synovial membrane at a lesion site of an articular rheumatism patient was subjected to tissue culture in an RPMI-1640 medium containing 10% fetal bovine serum by the method of Tanaka et al. (Tanaka M, Harigai M, Kawaguchi Y, Ohta S, Sugiura T, Takagi K, Ohsako-Higami S, Fukasawa C, Hara M, Kamatani N., J Rheumatol. 2001 Aug; 28(8):1779-87). The medium was exchanged with the aforementioned medium containing 100 nM of paraquat, which is an active oxygen generator, and the cells were cultured for 15 minutes, after which the medium was exchanged with a paraquat-free medium, and the cells were further cultured for 1 hour, after which the medium was collected, and the concentration of IL-18 protein in the medium was measured. Also, during paraquat treatment, the cells were cultured in a medium containing SOD (1, 2.5, 5, 10, 20 U/ml), which is an active oxygen eliminator, and the concentration of IL-18 protein in the medium was measured. In the synovial membrane tissue, the activation and release of IL-18 by the active oxygen molecular species were observed. Furthermore, these were suppressed by SOD, which is an active oxygen eliminator, or by vitamin E, which neutralizes active oxygen, dose-dependently (Figs. 11A and 11B).

### [Example 15]

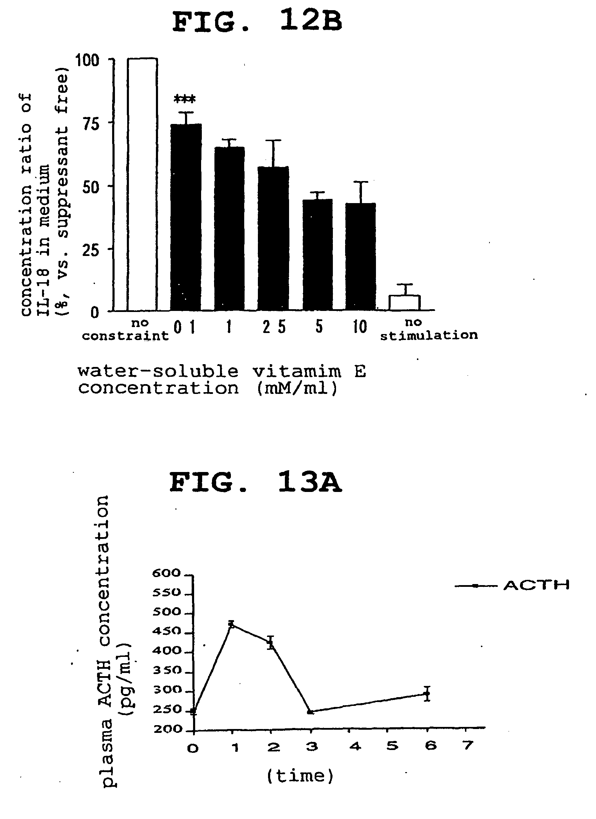

Primary culture human renal proximal tubular epithelial cells (purchased from CAMBREX Company) were cultured in a basal medium for renal tubular cells: contents 10 µg/ml hEGF (recombinant human epithelial cell growth factor) 0.5 ml, 5 mg/ml insulin 0.5 ml, 0.5 mg/ml hydrocortisone 0.5 ml, FBS (fetal bovine serum) 2.5 ml, 0.5 mg/ml epinephrine 0.5 ml, 6.5 µg/ml triiodothyronine 0.5 ml, 10 mg/ml transferrin 0.5 ml, 50 mg/ml gentamycin, 50 µg/ml amphotericin-B 0.5 ml), in petri dishes for cell culture 6 cm in diameter at 1.0-2.0 x 10⁶/dish. This was cultured in the aforementioned medium containing 100 nM of paraquat, which is an active oxygen generator, for 15 minutes, after which the medium was exchanged with the same but paraquat-free medium. After the cells were further cultured for 1 hour, the medium was collected, and the concentration of IL-18 protein in the medium was measured. Also, during paraquat treatment, the cells were cultured in a medium containing SOD (1, 2.5, 5, 10, 20 U/ml), which is an active oxygen eliminator, or water-soluble vitamin E (100 nM, 1, 2.5, 5, 10 mM/ml), and the concentration of IL-18 protein in the medium was measured. In the renal tubular cells, the activation and release of IL-18 by the active oxygen molecular species were observed. Furthermore, these were suppressed by SOD, which is an active oxygen eliminator, or by vitamin E, which neutralizes active oxygen, dose-dependently (Figs. 12A and 12B).

### [Example 16]

### IL-18 elicits the release of ACTH, FSH, and LH

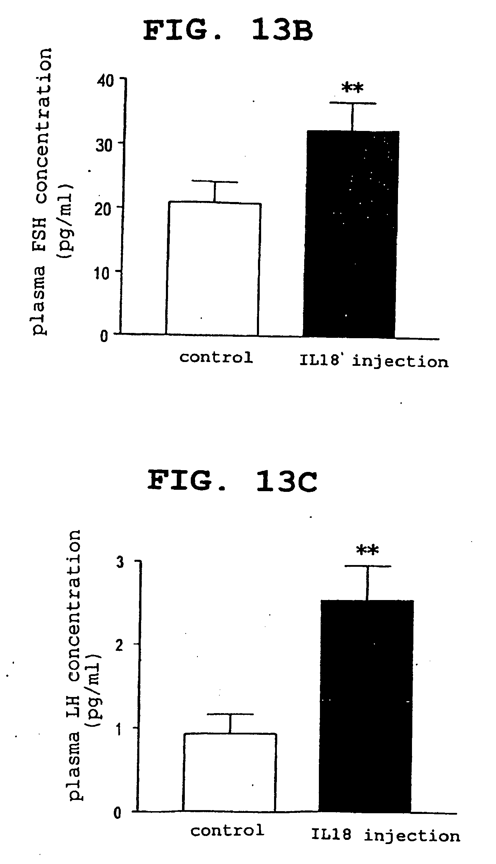

1.5 µg of IL-18 prepared by genetic recombination technology was intravenously injected to C57B/6 mice, and thereafter ACTH, FSH and LH levels in blood were examined. As shown in Fig. 13A, the blood ACTH concentration rose with the administration of IL-18. Figs. 13B and 13C show FSH and LH levels in plasma collected from mice 1 hour after IL-18 administration. It was shown that the concentrations of FSH and LH in plasma rose with the administration of IL-18. From this fact, it is understood that due to an elevation of IL-18 in blood, changes occur in the blood levels of ACTH, FSH, and LH, and it is found that an elevation of blood IL-18 due to stress and active oxygen significantly affects the systemic condition.

## Claims

1. A method of measuring the degree of stress excluding inflammatory stress, which comprises measuring an increase in the amount or activity of IL-18 and an increase in the activity of P38MAP kinase using an IL-18 antibody and a P38MAP kinase antibody as a visualizing agent, wherein the amount or activity of IL-18 is measured in adrenal tissue or blood collected from an animal and the activity of P38MAP kinase is measured in adrenal tissue collected from an animal.

2. The method of claim 1, wherein the IL-18 is active type IL-18 present in blood, the amount or activity of IL-18 in blood is measured and/or the P38MAP kinase is phosphorylated kinase.

3. The method of claim 2, wherein the P38MAP kinase is derived from a mouse, and the phosphorylation site of the kinase is Thr180 or Tyr182, or the P38MAP kinase is derived from a human, and the phosphorylation site of the kinase is Thr185 or Tyr187.

4. The method of any one of claims 1 to 3, wherein the stress is mental stress, optionally restraint stress.

5. The method of any one of the preceding claims, wherein the animal is a human.

6. Use of a visualizing agent which comprises an IL-18 antibody and a visualizing agent which comprises a P38MAP kinase antibody in detecting a response to stress excluding inflammatory stress by detecting an increase in the amount or activity of IL-18 and an increase in the activity of P38MAP kinase, wherein the amount or activity of IL-18 is detected in blood or adrenal tissue collected from an animal and the activity of P38MAP kinase is detected in adrenal tissue collected from an animal.

7. The use of claim 6, wherein the IL-18 antibody specifically recognizes active type IL-18 and/or the P38MAP kinase antibody is an anti-phosphorylated P38MAP kinase antibody.

8. The use of claim 7, wherein the anti-phosphorylated P38MAP kinase antibody recognizes a P38MAP kinase having phosphorylated Thr180 or Tyr182, or a P38MAP kinase having phosphorylated Thr185 or Tyr187.

9. Use of a kit in a method of measuring stress excluding inflammatory stress according to any one of claims 1 to 5, which kit comprises the visualizing agents of any one of claims 6 to 8.

10. A method of screening for a substance having a therapeutic effect, ameliorating effect or prophylactic effect on a disease that occurs, exacerbates or recurs due to mental stress, which comprises:
1) quantifying the amount or activity of IL-18 in adrenal tissue or blood collected from an animal and the activity of P38MAP kinase in adrenal tissue collected from the animal;
2) comparing the amount or activity of said IL-18 and the activity of said P38MAP kinase in the presence and absence of the test substance; and
3) judging that a test substance that reduces or increases the amount or activity of IL-18 and the activity of P38MAP kinase is a substance having a therapeutic effect, ameliorating effect or prophylactic effect on a disease that occurs, exacerbates or recurs due to mental stress, optionally wherein the disease is systemic lupus erythematosus, lupus nephritis, atopic dermatitis, multiple sclerosis, bronchial asthma, psoriasis, cerebral ischemia, cerebral degeneration, encephalitis, articular rheumatism, emmeniopathy, endometriosis or sexual desire reduction.

11. A method of screening for a substance having a suppressive effect or preventive effect on oxidative stress, which comprises:
1) quantifying the amount or activity of IL-18 in adrenal tissue or blood collected from an animal and the activity of P38MAP kinase in adrenal tissue collected from the animal;
2) comparing the amount or activity of said IL-18 and the activity of said P38MAP kinase in the presence and absence of the test substance; and
3) judging that a test substance that reduces or increases the amount or activity of IL-18 and the activity of P38MAP kinase is a substance having a suppressive effect or preventative effect on oxidative stress.

12. The method of claim 10 or 11, wherein the IL-18 is active-type IL-18 and/or the P38MAP kinase is phosphorylated kinase.

## Patentansprüche

1. Verfahren zum Messen des Ausmaßes von Stress mit Ausnahme von Entzündungsstress, umfassend das Messen einer Erhöhung der Menge oder Aktivität von IL-18 und einer Erhöhung der Aktivität von P38MAP-Kinase mit Hilfe eines IL-18-Antikörpers und eines P38MAP-Kinase-Antikörpers als Visualisierungsmittel, wobei die Menge oder Aktivität von IL-18 in Nebennierengewebe oder Blut, das von einem Tier gesammelt wird, gemessen wird und die Aktivität von P38MAP-Kinase in Nebennierengewebe, das von einem Tier gesammelt wird, gemessen wird.

2. Verfahren gemäß Anspruch 1, wobei es sich bei dem IL-18 um in Blut vorhandenes IL-18 des aktiven Typs handelt, die Menge oder Aktivität von IL-18 in Blut gemessen wird und/oder es sich bei der P38MAP-Kinase um phosphorylierte Kinase handelt.

3. Verfahren gemäß Anspruch 2, wobei die P38MAP-Kinase von einer Maus stammt und es sich bei der Phosphorylierungsstelle der Kinase um Thr180 oder Tyr182 handelt oder die P38MAP-Kinase von einem Menschen stammt und es sich bei der Phosphorylierungsstelle der Kinase um Thr185 oder Tyr187 handelt.

4. Verfahren gemäß einem der Ansprüche 1 bis 3, wobei es sich bei dem Stress um mentalen Stress, gegebenenfalls Immobilisierungsstress, handelt.

5. Verfahren gemäß einem der vorstehenden Ansprüche, wobei das Tier ein Mensch ist.

6. Verwendung eines Visualisierungsmittels, das einen IL-18-Antikörper umfasst, und eines Visualisierungsmittels, das einen P38MAP-Kinase-Antikörper umfasst, zum Nachweis einer Reaktion auf Stress mit Ausnahme von Entzündungsstress durch Nachweisen einer Erhöhung der Menge oder Aktivität von IL-18 und einer Erhöhung der Aktivität von P38MAP-Kinase, wobei die Menge oder Aktivität von IL-18 in Blut oder Nebennierengewebe, das von einem Tier gesammelt wird, gemessen wird und die Aktivität von P38MAP-Kinase in Nebennierengewebe, das von einem Tier gesammelt wird, gemessen wird.

7. Verwendung gemäß Anspruch 6, wobei der IL-18-Antikörper spezifisch IL-18 des aktiven Typs erkennt und/oder der P38MAP-Kinase-Antikörper ein Anti-phosphorylierte-P38MAP-Kinase-Antikörper ist.

8. Verwendung gemäß Anspruch 7, wobei der Anti-phosphorylierte-P38MAP-Kinase-Antikörper eine P38MAP-Kinase mit phosphoryliertem Thr180 oder Tyr182 oder eine P38MAP-Kinase mit phosphoryliertem Thr185 oder Tyr187 erkennt.

9. Verwendung eines Kits in einem Verfahren zum Messen von Stress mit Ausnahme von Entzündungsstress gemäß einem der Ansprüche 1 bis 5, wobei der Kit die Visualisierungsmittel gemäß einem der Ansprüche 6 bis 8 umfasst.

10. Verfahren zum Screening nach einer Substanz mit einer therapeutischen Wirkung, lindernden Wirkung oder prophylaktischen Wirkung auf eine Krankheit, die aufgrund von mentalem Stress auftritt, sich verschlimmert oder zurückkehrt, umfassend:
1) Quantifizieren der Menge oder Aktivität von IL-18 in Nebennierengewebe oder Blut, das von einem Tier gesammelt wird, und der Aktivität von P38MAP-Kinase in Nebennierengewebe, das von einem Tier gesammelt wird;
2) Vergleichen der Menge oder Aktivität des IL-18 und der Aktivität der P38MAP-Kinase in Anwesenheit und Abwesenheit der Testsubstanz; und
3) Beurteilen, dass eine Testsubstanz, die die Menge oder Aktivität von IL-18 und die Aktivität von P38MAP-Kinase reduziert oder erhöht, eine Substanz mit einer therapeutischen Wirkung, lindernden Wirkung oder prophylaktischen Wirkung auf eine Krankheit, die aufgrund von mentalem Stress auftritt, sich verschlimmert oder zurückkehrt, ist;
wobei es sich bei der Krankheit gegebenenfalls um systemischen Lupus erythematodes, Lupus nephritis, atopische Dermatitis, multiple Sklerose, Bronchialasthma, Psoriasis, Hirnblutung, Hirndegeneration, Enzephalitis, Gelenkrheumatismus, Menstruationsstörungen, Endometriose oder sexuelle Appetenzstörung handelt.

11. Verfahren zum Screening nach einer Substanz mit einer unterdrückenden Wirkung oder präventiven Wirkung auf oxidativen Stress, umfassend:
1) Quantifizieren der Menge oder Aktivität von IL-18 in Nebennierengewebe oder Blut, das von einem Tier gesammelt wird, und der Aktivität von P38MAP-Kinase in Nebennierengewebe, das von einem Tier gesammelt wird;
2) Vergleichen der Menge oder Aktivität des IL-18 und der Aktivität der P38MAP-Kinase in Anwesenheit und Abwesenheit der Testsubstanz; und
3) Beurteilen, dass eine Testsubstanz, die die Menge oder Aktivität von IL-18 und die Aktivität von P38MAP-Kinase reduziert oder erhöht, eine Substanz mit einer unterdrückenden Wirkung oder präventiven Wirkung auf oxidativen Stress ist.

12. Verfahren gemäß Anspruch 10 oder 11, wobei es sich bei dem IL-18 um IL-18 des aktiven Typs und/oder bei der P38MAP-Kinase um phosphorylierte Kinase handelt.

## Revendications

1. Procédé de mesure du degré de stress à l'exclusion du stress inflammatoire, qui comprend la mesure d'une hausse de la quantité ou de l'activité de l'IL-18 et une hausse de l'activité de la P38MAP kinase en utilisant un anticorps anti-IL-18 et un anticorps anti-P38MAP kinase en tant qu'agent de visualisation, dans lequel la quantité ou l'activité de l'IL-18 est mesurée dans les tissus des surrénales ou dans le sang prélevés chez un animal et l'activité de la P38MAP kinase est mesurée dans les tissus des surrénales prélevés chez un animal.

2. Procédé selon la revendication 1, dans lequel l'IL-18 est l'IL-18 de type actif, présente dans le sang, la quantité ou l'activité de l'IL-18 dans le sang étant mesurée et/ou la P38MAP kinase étant une kinase phosphorylée.

3. Procédé selon la revendication 2, dans lequel la P38MAP kinase est dérivée d'une souris, et le site de phosphorylation de la kinase est Thr180 ou Tyr 182, ou la P38MAP kinase est dérivée d'un être humain, et le site de phosphorylation de la kinase est Thr185 ou Tyr 187.

4. Procédé selon l'une quelconque des revendications 1 à 3, dans lequel le stress est un stress mental, éventuellement un stress de contrainte.

5. Procédé selon l'une quelconque des revendications précédentes, dans lequel l'animal est un être humain.

6. Utilisation d'un agent de visualisation qui comprend un anticorps anti-IL-18 et un agent de visualisation qui comprend un anticorps anti-P38MAP kinase pour la détection d'une réponse à un stress à l'exclusion d'un stress inflammatoire en détectant une hausse de la quantité ou de l'activité de l'IL-18 et une hausse de l'activité de la P38MAP kinase, dans laquelle la quantité ou l'activité de l'IL-18 est détectée dans le sang ou les tissus des surrénales prélevés chez un animal et l'activité de la P38MAP kinase est détectée dans les tissus des surrénales prélevés chez un animal.

7. Utilisation selon la revendication 6, dans laquelle l'anticorps anti-IL-18 reconnaît spécifiquement l'IL-18 de type actif et/ou l'anticorps anti-P38MAP kinase est un anticorps anti-P38MAP kinase phosphorylée.

8. Utilisation selon la revendication 7, dans laquelle l'anticorps anti-P38MAP kinase phosphorylée reconnaît une P38MAP kinase ayant des sites Thr180 ou Tyr182 phosphorylés, ou une P38MAP kinase ayant des sites Thr185 ou Tyr187 phosphorylés.

9. Utilisation d'un kit dans un procédé de mesure du stress à l'exclusion du stress inflammatoire selon l'une quelconque des revendications 1 à 5, ledit kit comprenant les agents de visualisation selon l'une quelconque des revendications 6 à 8.

10. Procédé de criblage d'une substance ayant un effet thérapeutique, améliorant l'effet ou ayant un effet prophylactique sur une maladie qui survient, s'exacerbe ou réapparaît du fait d'un stress mental, ledit procédé comprenant :
1) quantifier la quantité ou l'activité de l'IL-18 dans les tissus des surrénales ou dans le sang prélevés chez un animal et l'activité de la P38MAP kinase dans les tissus des surrénales prélevés chez l'animal ;
2) comparer la quantité ou l'activité de ladite IL-18 et l'activité de ladite P38MAP kinase en présence et en absence de la substance test ; et
3) évaluer si une substance test qui réduit ou augmente la quantité ou l'activité de l'IL-18 et l'activité de la P38MAP kinase est une substance ayant un effet thérapeutique, améliorant l'effet ou ayant un effet prophylactique sur une maladie qui survient, s'exacerbe ou réapparaît du fait d'un stress mental, la maladie étant éventuellement un lupus érythémateux systémique, une néphrite lupique, une dermatite atopique, une sclérose en plaques, un asthme bronchique, un psoriasis, une ischémie cérébrale, une dégénérescence cérébrale, une encéphalite, un rhumatisme articulaire, une emméniopathie, une endométriose ou une réduction du désir sexuel.

11. Procédé de criblage d'une substance ayant un effet suppresseur ou un effet préventif sur le stress oxydatif, ledit procédé comprenant :
1) quantifier la quantité ou l'activité de l'IL-18 dans les tissus des surrénales ou dans le sang prélevés chez un animal et l'activité de la P38MAP kinase dans les tissus des surrénales prélevés chez l'animal ;
2) comparer la quantité ou l'activité de ladite IL-18 et l'activité de ladite P38MAP kinase en présence et en absence de la substance test ; et
3) évaluer si une substance test qui réduit ou augmente la quantité ou l'activité de l'IL-18 et l'activité de la P38MAP kinase est une substance ayant un effet suppresseur ou préventif sur le stress oxydatif.

12. Procédé selon la revendication 10 ou 11, dans lequel l'IL-18 est une IL-18 de type actif et/ou la P38MAP kinase est une kinase phosphorylée.


### SEQUENCE LISTING

<110> Sekiyama, Atsuo
<120> An Indicator of Non-inflammatory Stress Response and Use Thereof
<130> PCT0523ZZ
<150> JP 2004-193712
   <151> 2004-6-30
<150> JP 2005-023978
   <151> 2005-1-31
<160> 10
<170> PatentIn version 3.2
<210> 1
   <211> 1145
   <212> DNA
   <213> Homo sapiens
<400> 1
   attctctccc cagcttgctg agccctttgc tcccctggcg actgcctgga cagtcagcaa     60
   ggaattgtct cccagtgcat tttgccctcc tggctgccaa ctctggctgc taaagcggct    120
   gccacctgct gcagtctaca cagcttcggg aagaggaaag gaacctcaga ccttccagat    180
   cgcttcctct cgcaacaaac tatttgtcgc aggaataaag atggctgctg aaccagtaga    240
   agacaattgc atcaactttg tggcaatgaa atttattgac aatacgcttt actttatagc    300
   tgaagatgat gaaaacctgg aatcagatta ctttggcaag cttgaatcta aattatcagt    360
   cataagaaat ttgaatgacc aagttctctt cattgaccaa ggaaatcggc ctctatttga    420
   agatatgact gattctgact gtagagataa tgcaccccgg accatattta ttataagtat    480
   gtataaagat agccagccta gaggtatggc tgtaactatc tctgtgaagt gtgagaaaat    540
   ttcaactctc tcctgtgaga acaaaattat ttcctttaag gaaatgaatc ctcctgataa    600
   catcaaggat acaaaaagtg acatcatatt ctttcagaga agtgtcccag gacatgataa    660
   taagatgcaa tttgaatctt catcatacga aggatacttt ctagcttgtg aaaaagagag    720
   agaccttttt aaactcattt tgaaaaaaga ggatgaattg ggggatagat ctataatgtt    780
   cactgttcaa aacgaagact agctattaaa atttcatgcc gggcgcagtg gctcacgcct    840
   gtaatcccag ccctttggga ggctgaggcg ggcagatcac cagaggtcag gtgttcaaga    900
   ccagcctgac caacatggtg aaacctcatc tctactaaaa atacaaaaaa ttagctgagt    960
   gtagtgacgc atgccctcaa tcccagctac tcaagaggct gaggcaggag aatcacttgc   1020
   actccggagg tagaggttgt ggtgagccga gattgcacca ttgcgctcta gcctgggcaa   1080
   caacagcaaa actccatctc aaaaaataaa ataaataaat aaacaaataa aaaattcata   1140
   atgtg                                                               1145
<210> 2
   <211> 193
   <212> PRT
   <213> Homo sapiens
<400> 2
   Met Ala Ala Glu Pro Val Glu Asp Asn Cys Ile Asn Phe Val Ala Met
   1               5                   10                  15
   Lys Phe Ile Asp Asn Thr Leu Tyr Phe Ile Ala Glu Asp Asp Glu Asn
               20                  25                  30
   Leu Glu Ser Asp Tyr Phe Gly Lys Leu Glu Ser Lys Leu Ser Val Ile
           35                  40                  45
   Arg Asn Leu Asn Asp Gln Val Leu Phe Ile Asp Gln Gly Asn Arg Pro
       50                  55                  60
   Leu Phe Glu Asp Met Thr Asp Ser Asp Cys Arg Asp Asn Ala Pro Arg
   65                  70                  75                  80
   Thr Ile Phe Ile Ile Ser Met Tyr Lys Asp Ser Gln Pro Arg Gly Met
                   85                  90                  95
   Ala Val Thr Ile Ser Val Lys Cys Glu Lys Ile Ser Thr Leu Ser Cys
               100                 105                 110
   Glu Asn Lys Ile Ile Ser Phe Lys Glu Met Asn Pro Pro Asp Asn Ile
           115                 120                 125
   Lys Asp Thr Lys Ser Asp Ile Ile Phe Phe Gln Arg Ser Val Pro Gly
       130                 135                 140
   His Asp Asn Lys Met Gln Phe Glu Ser Ser Ser Tyr Glu Gly Tyr Phe
   145                 150                 155                 160
   Leu Ala Cys Glu Lys Glu Arg Asp Leu Phe Lys Leu Ile Leu Lys Lys
                   165                 170                 175
   Glu Asp Glu Leu Gly Asp Arg Ser Ile Met Phe Thr Val Gln Asn Glu
               180                 185                 190
   Asp
<210> 3
   <211> 1514
   <212> DNA
   <213> Homo sapiens
<400> 3
   gcccctccct ccgcccgccc gccggcccgc ccgtcagtct ggcaggcagg caggcaatcg      60
   gtccgagtgg ctgtcggctc ttcagctctc ccgctcggcg tcttccttcc tcctcccggt     120
   cagcgtcggc ggctgcaccg gcggcggcgc agtccctgcg ggaggggcga caagagctga     180
   gcggcggccg ccgagcgtcg agctcagcgc ggcggaggcg gcggcggccc ggcagccaac     240
   atggcggcgg cggcggcggc gggcgcgggc ccggagatgg tccgcgggca ggtgttcgac     300
   gtggggccgc gctacaccaa cctctcgtac atcggcgagg gcgcctacgg catggtgtgc     360
   tctgcttatg ataatgtcaa caaagttcga gtagctatca agaaaatcag cccctttgag     420
   caccagacct actgccagag aaccctgagg gagataaaaa tcttactgcg cttcagacat     480
   gagaacatca ttggaatcaa tgacattatt cgagcaccaa ccatcgagca aatgaaagat     540
   gtatatatag tacaggacct catggaaaca gatctttaca agctcttgaa gacacaacac     600
   ctcagcaatg accatatctg ctattttctc taccagatcc tcagagggtt aaaatatatc     660
   cattcagcta acgttctgca ccgtgacctc aagccttcca acctgctgct caacaccacc 720
   tgtgatctca agatctgtga ctttggcctg gcccgtgttg cagatccaga ccatgatcac 780
   acagggttcc tgacagaata tgtggccaca cgttggtaca gggctccaga aattatgttg 840
   aattccaagg gctacaccaa gtccattgat atttggtctg taggctgcat tctggcagaa 900
   atgctttcta acaggcccat ctttccaggg aagcattatc ttgaccagct gaaccacatt 960
   ttgggtattc ttggatcccc atcacaagaa gacctgaatt gtataataaa tttaaaagct 1020
   aggaactatt tgctttctct tccacacaaa aataaggtgc catggaacag gctgttccca 1080
   aatgctgact ccaaagctct ggacttattg gacaaaatgt tgacattcaa cccacacaag 1140
   aggattgaag tagaacaggc tctggcccac ccatatctgg agcagtatta cgacccgagt 1200
   gacgagccca tcgccgaagc accattcaag ttcgacatgg aattggatga cttgcctaag 1260
   gaaaagctca aagaactaat ttttgaagag actgctagat tccagccagg atacagatct 1320
   taaatttgtc aggtacctgg agtttaatac agtgagctct agcaagggag gcgctgcctt 1380
   ttgtttctag aatattatgt tcctcaaggt ccattatttt gtattctttt ccaagctcct 1440
   tattggaagg tattttttta aatttagaat taaaaattat ttagaaaaaa aaaaaaaaaa 1500
   aaaaaaaaaa aaaa 1514
<210> 4
   <211> 360
   <212> PRT
   <213> Homo sapiens
<400> 4
<210> 5
   <211> 1340
   <212> DNA
   <213> Mus musculus
<400> 5
   gtgcgaaaga actgaggctt tttctcatgg ctgaaaacaa acaccctgac aaaccactta 60
   aggtgttgga acagctgggc aaagaagtcc ttacggagta cctagaaaaa ttagtacaaa 120
   gcaatgtact gaaattaaag gaggaagata aacaaaaatt taacaatgct gaacgcagtg 180
   acaagcgttg ggtttttgta gatgccatga aaaagaaaca cagcaaagta ggtgaaatgc 240
   ttctccagac attcttcagt gtggacccag gcagccacca tggtgaagct aatctggaaa 300
   tggaggaacc agaagaatca ttgaacactc tcaagctttg ttcccctgaa gagttcacaa 360
   ggctttgcag agaaaagaca caagaaattt acccaataaa ggaggccaat ggccgtacac 420
   gaaaggctct tatcatatgc aatacagagt tcaaacatct ctcactgagg tatggggcta 480
   actttgacat cattggtatg aaaggccttc ttgaagactt aggctacgat gtggtggtga 540
   aagaggagct tacagcagag ggcatggagt cagagatgaa agactttgct gcactctcag 600
   aacaccagac atcagacagc acattcctgg tgctaatgtc tcatggcaca ctgcatggca 660
   tttgtggaac aatgcacagt gaaaaaactc cagatgtgct acagtatgat accatctatc 720
   agatattcaa caattgccac tgtccaggtc tacgagacaa acccaaagtc atcattgtgc 780
   aggcctgcag aggtgggaac tctggagaaa tgtggatcag agagtcttca aaaccccagt 840
   tgtgcagagg tgtagatcta cctaggaata tggaagctga tgctgtcaag ctgagccacg 900
   tggagaagga cttcattgcc ttctactcta caaccccaca tcacttgtcc taccgagaca 960
   aaacaggagg ctcttacttc atcactagac tcatttcctg cttccggaaa catgcttgct 1020
   cttgtcatct ctttgatata ttcctgaagg tgcaacaatc atttgaaaag gcaagtattc 1080
   attcccagat gcccaccatt gatcgggcaa ccttgacgag atatttctac ctctttcctg 1140
   gcaactgaga acaaagcaac aagcaactga atctcatttc ttcagcttga agaagtgatc 1200
   ttggccaagg atcacattct attcctgaaa ttccagaact agtgaaatta aggaaagaat 1260
   acttatgaat tcaagaccag cctaagcaac acagtgggat tctgttccat agacaagcaa 1320
   acaagcaaaa ataaaaaaaa 1340
<210> 6
   <211> 373
   <212> PRT
   <213> Mus musculus
<400> 6
   Met Ala Glu Asn Lys His Pro Asp Lys Pro Leu Lys Val Leu Glu Gln
   1 5 10 15
   Leu Gly Lys Glu Val Leu Thr Glu Tyr Leu Glu Lys Leu Val Gln Ser
   20 25 30
   Asn Val Leu Lys Leu Lys Glu Glu Asp Lys Gln Lys Phe Asn Asn Ala
   35 40 45
   Glu Arg Ser Asp Lys Arg Trp Val Phe Val Asp Ala Met Lys Lys Lys
   50 55 60
   His Ser Lys Val Gly Glu Met Leu Leu Gln Thr Phe Phe Ser Val Asp
   65 70 75 80
   Pro Gly Ser His His Gly Glu Ala Asn Leu Glu Met Glu Glu Pro Glu
   85 90 95
   Glu Ser Leu Asn Thr Leu Lys Leu Cys Ser Pro Glu Glu Phe Thr Arg
   100 105 110
   Leu Cys Arg Glu Lys Thr Gln Glu Ile Tyr Pro Ile Lys Glu Ala Asn
   115 120 125
   Gly Arg Thr Arg Lys Ala Leu Ile Ile Cys Asn Thr Glu Phe Lys His
   130 135 140
   Leu Ser Leu Arg Tyr Gly Ala Asn Phe Asp Ile Ile Gly Met Lys Gly
   145 150 155 160
   Leu Leu Glu Asp Leu Gly Tyr Asp Val Val Val Lys Glu Glu Leu Thr
   165 170 175
   Ala Glu Gly Met Glu Ser Glu Met Lys Asp Phe Ala Ala Leu Ser Glu
   180 185 190
   His Gln Thr Ser Asp Ser Thr Phe Leu Val Leu Met Ser His Gly Thr
   195 200 205
   Leu His Gly Ile Cys Gly Thr Met His Ser Glu Lys Thr Pro Asp Val
   210 215 220
   Leu Gln Tyr Asp Thr Ile Tyr Gln Ile Phe Asn Asn Cys His Cys Pro
   225 230 235 240
   Gly Leu Arg Asp Lys Pro Lys Val Ile Ile Val Gln Ala Cys Arg Gly
   245 250 255
   Gly Asn Ser Gly Glu Met Trp Ile Arg Glu Ser Ser Lys Pro Gln Leu
   260 265 270
   Cys Arg Gly Val Asp Leu Pro Arg Asn Met Glu Ala Asp Ala Val Lys
   275 280 285
   Leu Ser His Val Glu Lys Asp Phe Ile Ala Phe Tyr Ser Thr Thr Pro
   290 295 300
   His His Leu Ser Tyr Arg Asp Lys Thr Gly Gly Ser Tyr Phe Ile Thr
   305 310 315 320
   Arg Leu Ile Ser Cys Phe Arg Lys His Ala Cys Ser Cys His Leu Phe
   325 330 335
   Asp Ile Phe Leu Lys Val Gln Gln Ser Phe Glu Lys Ala Ser Ile His
   340 345 350
   Ser Gln Met Pro Thr Ile Asp Arg Ala Thr Leu Thr Arg Tyr Phe Tyr
   355 360 365
   Leu Phe Pro Gly Asn
   370
<210> 7
   <211> 1373
   <212> DNA
   <213> Homo sapiens
<400> 7
   gggaggagag aaaagccatg gccgacaagg tcctgaagga gaagagaaag ctgtttatcc 60
   gttccatggg tgaaggtaca ataaatggct tactggatga attattacag acaagggtgc 120
   tgaacaagga agagatggag aaagtaaaac gtgaaaatgc tacagttatg gataagaccc 180
   gagctttgat tgactccgtt attccgaaag gggcacaggc atgccaaatt tgcatcacat 240
   acatttgtga agaagacagt tacctggcag ggacgctggg actctcagca gatcaaacat 300
   ctggaaatta ccttaatatg caagactctc aaggagtact ttcttccttt ccagctcctc 360
   aggcagtgca ggacaaccca gctatgccca catcctcagg ctcagaaggg aatgtcaagc    420
   tttgctccct agaagaagct caaaggatat ggaaacaaaa gtcggcagag atttatccaa    480
   taatggacaa gtcaagccgc acacgtcttg ctctcattat ctgcaatgaa gaatttgaca    540
   gtattcctag aagaactgga gctgaggttg acatcacagg catgacaatg ctgctacaaa    600
   atctggggta cagcgtagat gtgaaaaaaa atctcactgc ttcggacatg actacagagc    660
   tggaggcatt tgcacaccgc ccagagcaca agacctctga cagcacgttc ctggtgttca    720
   tgtctcatgg tattcgggaa ggcatttgtg ggaagaaaca ctctgagcaa gtcccagata    780
   tactacaact caatgcaatc tttaacatgt tgaataccaa gaactgccca agtttgaagg    840
   acaaaccgaa ggtgatcatc atccaggcct gccgtggtga cagccctggt gtggtgtggt    900
   ttaaagattc agtaggagtt tctggaaacc tatctttacc aactacagaa gagtttgagg    960
   atgatgctat taagaaagcc cacatagaga aggattttat cgctttctgc tcttccacac   1020
   cagataatgt ttcttggaga catcccacaa tgggctctgt ttttattgga agactcattg   1080
   aacatatgca agaatatgcc tgttcctgtg atgtggagga aattttccgc aaggttcgat   1140
   tttcatttga gcagccagat ggtagagcgc agatgcccac cactgaaaga gtgactttga   1200
   caagatgttt ctacctcttc ccaggacatt aaaataagga aactgtatga atgtctgtgg   1260
   gcaggaagtg aagagatcgt tctgtaaaag gtttttggaa ttatgtctgc tgaataataa   1320
   actttttttg aaataataaa tctggtagaa aaatgaaaaa aaaaaaaaaa aaa          1373
<210> 8
   <211> 404
   <212> PRT
   <213> Homo sapiens
<400> 8
   Met Ala Asp Lys Val Leu Lys Glu Lys Arg Lys Leu Phe Ile Arg Ser
   1               5                   10                  15
   Met Gly Glu Gly Thr Ile Asn Gly Leu Leu Asp Glu Leu Leu Gln Thr
               20                  25                  30
   Arg Val Leu Asn Lys Glu Glu Met Glu Lys Val Lys Arg Glu Asn Ala
           35                  40                  45
   Thr Val Met Asp Lys Thr Arg Ala Leu Ile Asp Ser Val Ile Pro Lys
       50                  55                  60
   Gly Ala Gln Ala Cys Gln Ile Cys Ile Thr Tyr Ile Cys Glu Glu Asp
   65                  70                  75                  80
   Ser Tyr Leu Ala Gly Thr Leu Gly Leu Ser Ala Asp Gln Thr Ser Gly
                   85                  90                  95
   Asn Tyr Leu Asn Met Gln Asp Ser Gln Gly Val Leu Ser Ser Phe Pro
               100                 105                 110
   Ala Pro Gln Ala Val Gln Asp Asn Pro Ala Met Pro Thr Ser Ser Gly
           115                 120                 125
   Ser Glu Gly Asn Val Lys Leu Cys Ser Leu Glu Glu Ala Gln Arg Ile
       130                 135                 140
   Trp Lys Gln Lys Ser Ala Glu Ile Tyr Pro Ile Met Asp Lys Ser Ser
   145 150 155 160
   Arg Thr Arg Leu Ala Leu Ile Ile Cys Asn Glu Glu Phe Asp Ser Ile
   165 170 175
   Pro Arg Arg Thr Gly Ala Glu Val Asp Ile Thr Gly Met Thr Met Leu
   180 185 190
   Leu Gln Asn Leu Gly Tyr Ser Val Asp Val Lys Lys Asn Leu Thr Ala
   195 200 205
   Ser Asp Met Thr Thr Glu Leu Glu Ala Phe Ala His Arg Pro Glu His
   210 215 220
   Lys Thr Ser Asp Ser Thr Phe Leu Val Phe Met Ser His Gly Ile Arg
   225 230 235 240
   Glu Gly Ile Cys Gly Lys Lys His Ser Glu Gln Val Pro Asp Ile Leu
   245 250 255
   Gln Leu Asn Ala Ile Phe Asn Met Leu Asn Thr Lys Asn Cys Pro Ser
   260 265 270
   Leu Lys Asp Lys Pro Lys Val Ile Ile Ile Gln Ala Cys Arg Gly Asp
   275 280 285
   Ser Pro Gly Val Val Trp Phe Lys Asp Ser Val Gly Val Ser Gly Asn
   290 295 300
   Leu Ser Leu Pro Thr Thr Glu Glu Phe Glu Asp Asp Ala Ile Lys Lys
   305 310 315 320
   Ala His Ile Glu Lys Asp Phe Ile Ala Phe Cys Ser Ser Thr Pro Asp
   325 330 335
   Asn Val Ser Trp Arg His Pro Thr Met Gly Ser Val Phe Ile Gly Arg
   340 345 350
   Leu Ile Glu His Met Gln Glu Tyr Ala Cys Ser Cys Asp Val Glu Glu
   355 360 365
   Ile Phe Arg Lys Val Arg Phe Ser Phe Glu Gln Pro Asp Gly Arg Ala
   370 375 380
   Gln Met Pro Thr Thr Glu Arg Val Thr Leu Thr Arg Cys Phe Tyr Leu
   385 390 395 400
   Phe Pro Gly His
<210> 9
   <211> 1125
   <212> DNA
   <213> Homo sapiens
<400> 9
   ttctgccctc gagcccaccg ggaacgaaag agaagctcta tctcgcctcc aggagcccag 60
   ctatgaactc cttctccaca agcgccttcg gtccagttgc cttctccctg gggctgctcc 120
   tggtgttgcc tgctgccttc cctgccccag tacccccagg agaagattcc aaagatgtag 180
   ccgccccaca cagacagcca ctcacctctt cagaacgaat tgacaaacaa attcggtaca 240
   tcctcgacgg catctcagcc ctgagaaagg agacatgtaa caagagtaac atgtgtgaaa 300
   gcagcaaaga ggcactggca gaaaacaacc tgaaccttcc aaagatggct gaaaaagatg 360
   gatgcttcca atctggattc aatgaggaga cttgcctggt gaaaatcatc actggtcttt 420
   tggagtttga ggtataccta gagtacctcc agaacagatt tgagagtagt gaggaacaag 480
   ccagagctgt gcagatgagt acaaaagtcc tgatccagtt cctgcagaaa aaggcaaaga 540
   atctagatgc aataaccacc cctgacccaa ccacaaatgc cagcctgctg acgaagctgc 600
   aggcacagaa ccagtggctg caggacatga caactcatct cattctgcgc agctttaagg 660
   agttcctgca gtccagcctg agggctcttc ggcaaatgta gcatgggcac ctcagattgt 720
   tgttgttaat gggcattcct tcttctggtc agaaacctgt ccactgggca cagaacttat 780
   gttgttctct atggagaact aaaagtatga gcgttaggac actattttaa ttatttttaa 840
   tttattaata tttaaatatg tgaagctgag ttaatttatg taagtcatat ttatattttt 900
   aagaagtacc acttgaaaca ttttatgtat tagttttgaa ataataatgg aaagtggcta 960
   tgcagtttga atatcctttg tttcagagcc agatcatttc ttggaaagtg taggcttacc 1020
   tcaaataaat ggctaactta tacatatttt taaagaaata tttatattgt atttatataa 1080
   tgtataaatg gtttttatac caataaatgg cattttaaaa aattc 1125
<210> 10
   <211> 212
   <212> PRT
   <213> Homo sapiens
<400> 10
   Met Asn Ser Phe Ser Thr Ser Ala Phe Gly Pro Val Ala Phe Ser Leu
   1 5 10 15
   Gly Leu Leu Leu Val Leu Pro Ala Ala Phe Pro Ala Pro Val Pro Pro
   20 25 30
   Gly Glu Asp Ser Lys Asp Val Ala Ala Pro His Arg Gln Pro Leu Thr
   35 40 45
   Ser Ser Glu Arg Ile Asp Lys Gln Ile Arg Tyr Ile Leu Asp Gly Ile
   50 55 60
   Ser Ala Leu Arg Lys Glu Thr Cys Asn Lys Ser Asn Met Cys Glu Ser
   65 70 75 80
   Ser Lys Glu Ala Leu Ala Glu Asn Asn Leu Asn Leu Pro Lys Met Ala
   85 90 95
   Glu Lys Asp Gly Cys Phe Gln Ser Gly Phe Asn Glu Glu Thr Cys Leu
   100 105 110
   Val Lys Ile Ile Thr Gly Leu Leu Glu Phe Glu Val Tyr Leu Glu Tyr
   115 120 125
   Leu Gln Asn Arg Phe Glu Ser Ser Glu Glu Gln Ala Arg Ala Val Gln
   130 135 140
   Met Ser Thr Lys Val Leu Ile Gln Phe Leu Gln Lys Lys Ala Lys Asn
   145 150 155 160
   Leu Asp Ala Ile Thr Thr Pro Asp Pro Thr Thr Asn Ala Ser Leu Leu
   165 170 175
   Thr Lys Leu Gln Ala Gln Asn Gln Trp Leu Gln Asp Met Thr Thr His
   180 185 190
   Leu Ile Leu Arg Ser Phe Lys Glu Phe Leu Gln Ser Ser Leu Arg Ala
   195 200 205
   Leu Arg Gln Met
   210